# EUROPEAN PATENT APPLICATION

(11) **EP 4 578 545 A1**
(43) Date of publication of application: **02.07.2025**
(21) Application number: 23856722.6
(22) Date of filing: 25.08.2023
(51) Int. Cl.: B01J 29/70, B01J 29/18, B01J 29/08, C07C 2/66, C07C 15/107

(54) **METAL-MODIFIED MOLECULAR SIEVE, AND PREPARATION AND USE THEREOF**

(30) Priority: 26.08.2022 CN 202211033764; 26.08.2022 CN 202211035148; 26.08.2022 CN 202211033765
(71) Applicant: China Petroleum & Chemical Corporation, Beijing 100728 (CN); Sinopec Research Institute of Petroleum Processing Co., Ltd., Beijing 100083 (CN)
(72) Inventor: ZHANG, Chengxi, Beijing 100083 (CN); LI, Yongxiang, Beijing 100083 (CN); ZHOU, Shunli, Beijing 100083 (CN); FU, Qiang, Beijing 100083 (CN)
(74) Representative: Hoffmann Eitle
(86) International application number: PCT/CN2023/114925
(87) International publication number: WO 2024/041636

(57) **Abstract**

A metal-modified molecular sieve, preparation and use thereof are disclosed. The metal-modified molecular sieve comprises a silica-alumina molecular sieve and a modifying metal selected from an alkaline earth metal, a Group IIIB metal, a Group IIIA metal, or combinations thereof, wherein at least a portion of the modifying metal exists in the form of an extra-framework compensation cation of the molecular sieve, and the modifying metal as element represents from 8 wt.% to 30 wt.% based on the total amount of the metal-modified molecular sieve; in the metal-modified molecular sieve, the ratio of the weight percentage of the surface-layer modifying metal measured by X-ray photoelectron spectroscopy to the weight percentage of the bulk modifying metal measured by X-ray fluorescence does not exceed 1.45, calculated on the basis of the weight percentage of the element. When being used in the preparation of a long-chain hydrocarbon-based aromatic compound, the molecular sieve can effectively inhibit the skeletal isomerization of the target long-chain hydrocarbon-based product, improve the product linearity and the product selectivity, and simultaneously, its catalytic cycle life is also greatly extended.

## Description

### Technical Field

The present application relates to the field of catalysts, specifically to a metal-modified molecular sieve, and preparation and use thereof.

### Background of Art

Long-chain alkylbenzenes are imporant raw materials for producing a synthetic detergent, sodium alkylbenzene sulfonates. Long-chain alkylbenzenes are mainly prepared by the alkylation reaction of long-chain olefins with monocyclic aromatic hydrocarbons. The current commercial process technology mainly uses hydrofluoric acid or aluminum chloride as a catalyst, but these two catalytic systems have great safety and environmental risks and are gradually eliminated as safety and environmental requirements gradually increase. The development of a new non-corrosive and non-polluting solid acid alkylation method as an alternative technology has become an inevitable development trend in the production technology of long-chain alkylbenzenes.

Currently, a fluorine-containing SiO₂-Al₂O₃ solid acid catalyst (ZL93104573.8), jointly developed by UOP of the United States and Petresa of Spain, is utilized in industrialized solid acid processes. The catalyst needs to be periodically flushed with benzene to restore its activity in order to achieve long-term stable operation. Simultaneously, since the catalyst itself contains fluorine, it is corrosive to the equipment and still has certain safety and environmental risks.

The catalyst system of zeolite molecular sieves has been widely studied domestically and abroad. Since long-chain alkylbenzene molecules are relatively large, they cannot enter channels of ten-membered rings or below. Only molecular sieves having channels of twelve-membered rings or above, such as MOR, Y, and Beta, have good catalytic performance for the alkylation reaction of benzene with long-chain olefins (Catal Surv Asia (2014) 18: 1-12; Catal Today (2017) 298: 109-116). On the other hand, since branched alkylbenzenes are not readily degradable, the national standard has strict requirements on linearity of the alkylbenzene products. The primary challenges in the synthesis of long-chain alkylbenzenes under the catalysis of molecular sieve are short catalyst lifetime and low linearity.

CN107867699A discloses a Y zeolite containing regular ultra-large micropores. By subjecting the selected Y zeolite to template and acid-base treatment, a Y zeolite with regular ultra-large micropores of 1-2 nm is constructed. Due to the abundant acid centers and suitable reaction channels provided by the regular ultra-large micropores, a higher conversion rate and selectivity are achieved.

CN110562995A discloses a method for synthesizing a nano Y zeolite and use in the synthesis of linear alkylbenzenes. Due to the catalyst grains, the diffusion resistance is low, and the activity and stability of long-chain olefin alkylation are improved.

CN102639471A discloses a method for producing a linear alkylbenzene, which uses a combination of two zeolites to limit skeletal isomerization to increase the product linearity.

However, in the prior art, in the preparation of long-chain alkylbenzenes, due to the relatively high length of long-chain olefins, the activation and skeletal isomerization of long-chain olefins still pose great challenges to the activity and selectivity of the reaction. On the other hand, the catalytic cycle life of the catalyst in the prior art is still short, the product linearity is low, and the preparation process is long and the cost is high.

### Summary of the Invention

The purpose of the present application is to provide a metal-modified molecular sieve, and preparation and use thereof. The metal-modified molecular sieve contains a specific amount of extra-framework modifying metal, which can effectively inhibit the skeletal isomerization of long-chain hydrocarbon-based aromatic compound products, improve the product linearity and the product selectivity, and simultaneously its catalytic cycle life is also greatly extended.

In order to achieve the above object, on the one hand, the present application provides a metal-modified molecular sieve, comprising a silica-alumina molecular sieve and a modifying metal selected from an alkaline earth metal, a Group IIIB metal, a Group IIIA metal, or combinations thereof, wherein at least a portion of the modifying metal exists in the form of an extra-framework compensation cation of the molecular sieve, and the content of the modifying metal as element ranges from 8 wt.% to 30 wt.% based on the total amount of the metal-modified molecular sieve;
wherein, in the metal-modified molecular sieve, the ratio of the weight percentage of the surface-layer modifying metal measured by X-ray photoelectron spectroscopy (XPS) to the weight percentage of the bulk modifying metal measured by X-ray fluorescence (XRF) does not exceed 1.45, calculated on the basis of the weight percentage of the element.

On the other hand, a method for preparing the metal-modified molecular sieve of the present application is provided, comprising the following steps:
1) performing a first ion exchange of a molecular sieve precursor with a first exchange liquid containing a soluble compound of modifying metal;
2) subjecting the product of the first ion exchange to a first calcination to obtain a first exchanged molecular sieve;
3) performing a second ion exchange of the first exchanged molecular sieve with a second exchange liquid containing a soluble compound of modifying metal;
4) subjecting the product of the second ion exchange to a second calcination, and optionally repeating the second ion exchange and the second calcination one or more times to obtain a second exchanged molecular sieve;
5) heat-treating the second exchanged molecular sieve in an oxygen-containing atmosphere to obtain the metal-modified molecular sieve;
wherein, the first calcination and/or the second calcination is/are performed under an alkaline atmosphere.

On the other hand, a solid acid catalyst is provided, comprising the metal-modified molecular sieve of the present application and a heat-resistant inorganic oxide.

On the other hand, use of the metal-modified molecular sieve and the solid acid catalyst of the present application in the preparation of a long-chain hydrocarbon-based aromatic compound is provided.

On the other hand, a method for preparing a long-chain hydrocarbon-based aromatic compound is provided, comprising a step of reacting a long-chain olefin and an aromatic hydrocarbon in the presence of the metal-modified molecular sieve of the present application.

Preferably, the method is carried out in the presence of the solid acid catalyst of the present application, and the method further comprises a step of regenerating the solid acid catalyst in the presence of an oxygen-containing mixed gas.

By means of the modification with a certain amount of extra-framework metal with a specific distribution in the surface layer and channels of the molecular sieve, the metal-modified molecular sieve provided by the present application can effectively inhibit the skeletal isomerization of the target long-chain hydrocarbon-based product in the preparation process of a long-chain hydrocarbon-based aromatic compound, and improve the product linearity and the product selectivity, and simultaneously, its catalytic cycle life is also greatly extended.

The method for preparing the metal-modified molecular sieve provided by the present application can achieve the modification with a specific amount of extra-framework metal with a specific distribution in the surface layer and channels of the molecular sieve through multiple ion exchanges and multiple calcinations, while ensuring the stability of the unit cell size of the molecular sieve before and after the reaction, and avoiding the destruction of the molecular sieve structure due to multiple calcinations.

The solid acid catalyst provided by the present application can achieve the shape selectivity of the reaction product in the preparation process of a long-chain alkylbenzene by the coordination of the metal-modified molecular sieve and the heat-resistant inorganic oxide, so that the product linearity is significantly improved and the product selectivity is improved; simultaneously, the solid acid catalyst provided by the present application is applicable to various forms of reactors such as fixed bed, fluidized bed and moving bed in the industrial production, and a large-scale of the industrial application can be achieved.

The method for preparing the long-chain hydrocarbon-based aromatic compound provided by the present application uses the metal-modified molecular sieve and/or the solid acid catalyst. Due to the shape selectivity of the reaction product after the modification of the molecular sieve, the product linearity is significantly improved, and the product selectivity is improved; and simultaneously, the catalytic cycle life of the metal-modified molecular sieve is also significantly extended. When the solid acid catalyst of the present application is used, the catalyst can also be regenerated conveniently, and a long-term continuous operation can be achieved through reaction-regeneration, thereby achieving the continuous and stable preparation of long-chain hydrocarbon-based aromatic compounds, especially long-chain alkylbenzenes.

Other features and advantages of the present application will be described in detail in the subsequent detailed description.

### Brief description of the drawings

The drawings are intended to provide a further understanding of the present application and form a part of the specification. They are used together with the detailed description below to explain the present application but do not constitute a limitation on the present application. In the figures:
Figure 1 shows a comparison of the metal-modified molecular sieve obtained in Inventive Preparation Example I-1 and an HY molecular sieve by XRD spectra;
Figure 2 shows the pyridine adsorption infrared spectra of the molecular sieves obtained in Inventive Preparation Example I-1 and Comparative Preparation Examples I-1 to I-3;
Figure 3 shows a comparison of the metal-modified molecular sieve obtained in Inventive Preparation Example I-2 and an Hβ molecular sieve by XRD spectra;
Figure 4 shows an evaluation result of a 300 h continuous operation in the preparation of linear long-chain alkylbenzenes with the metal-modified molecular sieve obtained in Inventive Preparation Example I-1;
Figure 5 shows an evaluation result of a 260 h continuous operation in the preparation of linear long-chain alkylbenzenes with the solid acid catalyst obtained in Inventive Preparation Example II-1; and
Figure 6 shows an evaluation result of a continuous preparation of linear long-chain alkylbenzenes in a continuous reaction-regeneration manner with the solid acid catalyst obtained in Inventive Preparation Example II-1.

### Detailed description

The following provides a detailed explanation of specific embodiments of this application in conjunction with the drawings. It should be understood that the specific embodiments described herein are only intended to illustrate and explain the present application, and are not intended to limit the present application.

Any specific numerical value disclosed herein (including the endpoints of a numerical range) is not limited to the precise value of the numerical value, but is to be understood to further cover values close to the precise value, e.g. all possible values within ±5% of the precise value. Moreover, for the disclosed numerical ranges, one or more new numerical ranges can be obtained by any combination between the endpoint values of the ranges, between one endpoint value of the ranges and one specific point value within the ranges, and between any two specific point values of the ranges. These new numerical ranges shall also be deemed to be specifically disclosed herein.

Unless otherwise stated, the terms used herein have the same meanings as commonly understood by those skilled in the art. If a term is defined herein and its definition is different from the common understanding in the art, the definition herein shall prevail.

In the present application, the term "extra-framework (...) of the molecular sieve" refers to the modified metal being present in the form of ions in the cationic position of the molecular sieve and/or being loaded in the form of oxides on the surface of the molecular sieve. The peak position of the characteristic peaks of the XRD test of the metal-modified molecular sieve is identical to the peak position of the diffraction peaks of the unmodified molecular sieve, that is, there is no shift of the characteristic peaks, which proves that the modifying metals are all extra-framework of the molecular sieve.

In the present application, the weight percentage of the surface-layer modifying metal refers to the weight percentage of the modifying metal as element measured by XPS, and the XPS detection depth is in the range from 0 to 10 nm from the surface of the molecular sieve. The weight percentage of the bulk modifying metal refers to the weight percentage of the modifying metal as element, which is obtained by the measurement with XRF and further the calculation. It can be understood that the weight percentage of the bulk modifying metal as oxide is firstly obtained by the XRF test, and then the weight percentage of the corresponding element is obtained through the calculation.

In the present application, the "dry basis weight" is defined as the weight of the material after calcination at 600°C for 3 hours.

In this application, unless expressly stated, any issues or matters not mentioned shall directly apply to those known in the art without any change. Moreover, any embodiment described herein can be freely combined with one or more other embodiments described herein, and the technical solutions or technical ideas thus formed shall be regarded as a part of those originally disclosed or recorded in this application and shall not be considered to be new matter that has not been disclosed or expected herein, unless those skilled in the art believe that the combination is obviously unreasonable.

All patent literatures and non-patent literatures including but not limited to textbooks and journal articles mentioned herein are incorporated herein by reference in their entirety.

In a first aspect, the present application provides a metal-modified molecular sieve, comprising a silica-alumina molecular sieve and a modifying metal selected from an alkaline earth metal, a Group IIIB metal, a Group IIIA metal, or combinations thereof, wherein at least a portion of the modifying metal exists in the form of an extra-framework compensation cation of the molecular sieve, and the content of the modifying metal as element ranges from 8 wt.% to 30 wt.%, based on the total amount of the metal-modified molecular sieve;
wherein, in the metal-modified molecular sieve, the ratio of the weight percentage of the surface-layer modifying metal measured by X-ray photoelectron spectroscopy (XPS) to the weight percentage of the bulk modifying metal measured by X-ray fluorescence spectroscopy (XRF) does not exceed 1.45, calculated on the basis of the weight percentage of the element.

After the extensive research, the inventors of the present application have discovered that when a certain amount of extra-framework modifying metal is contained inside the molecular sieve and the modifying metal has a specific distribution in the surface layer and channels of the molecular sieve, its use in the alkylation reaction of a long-chain olefin with an aromatic hydrocarbon can effectively inhibit the occurrence of side reactions such as olefin isomerization and polyalkylation reactions that lead to the generation of by-products and the catalyst deactivation, thereby achieving the shape-selective catalysis and improving the product linearity and the catalytic cycle life of the molecular sieve.

In a preferred embodiment, in the metal-modified molecular sieve, the ratio of the weight percentage of the surface-layer modifying metal measured by XPS to the weight percentage of the bulk modifying metal measured by XRF does not exceed 1.35, preferably ranges from 0.9 to 1.35, calculated on the basis of the weight percentage of the element, for example, it can be 0.95, 1, 1.05, 1.1, 1.15, 1.2, 1.25, 1.3, 1.35 or any numerical value within a numerical range formed by any two points. Under the above preferred distribution, it is beneficial to improve the catalytic cycle life of the molecular sieve and the product linearity. If the ratio of the weight percentage of the surface-layer modifying metal to the weight percentage of the bulk modifying metal is too high, the catalytic cycle life of the molecular sieve may be shortened, and if the ratio is too low, the product linearity may be reduced.

In a preferred embodiment, based on the total amount of the metal-modified molecular sieve, the content of the modifying metal as element ranges from 12 wt.% to 23 wt.%. For example, the content of the modifying metal as element can be 12 wt.%, 13 wt.%, 14 wt.%, 15 wt.%, 16 wt.%, 17 wt.%, 18 wt.%, 19 wt.%, 20 wt.%, 21 wt.%, 22 wt.%, 23 wt.% or any numerical value within a numerical range formed by any two points. In the above preferred case, it is beneficial to ensure that the metal distribution is in the optimum position, thereby achieving shape-selective catalysis, and improving the catalytic cycle life of the catalyst and the product linearity.

In a preferred embodiment, the modifying metal has an atomic radius ranging from 120 pm to 270 pm, and preferably from 160 pm to 240 pm. Further preferably, the modifying metal is selected from Mg, Ca, Sr, Ba, La, Ce, Pr, Rd, Sm, Eu, Yb, Sc, Y, Al, Ga, In, or combinations thereof; still further preferably La, Ce, Sr, Y, or combinations thereof. The use of the above preferred metals is conducive to matching with the channels of the molecular sieve, achieving the optimal shape-selective catalytic effect, protecting the structure of the molecular sieve, and improving its catalytic cycle life and the product linearity.

In a preferred embodiment, the molar ratio of silica/alumina ranges from 1:1 to 100:1, and further preferably from 3:1 to 20:1 in the metal-modified molecular sieve.

In a preferred embodiment, the metal-modified molecular sieve is selected from X-type molecular sieve, Y-type molecular sieve, MCM-22-type molecular sieve, Beta-type molecular sieve, MOR-type molecular sieve, or combinations thereof, preferably X-type molecular sieve and/or Y-type molecular sieve. The use of the above preferred type of the molecular sieve is conducive to further improving the catalytic activity of the molecular sieve.

In a preferred embodiment, the metal-modified molecular sieve has two absorption peaks in the range from 1440 cm⁻¹ to 1460 cm⁻¹ of the pyridine adsorption infrared spectrum. Further preferably, the ratio of the area of the absorption peak at the lower wavenumber position to the total area of the two absorption peaks ranges from 0.3 to 0.7, and more preferably from 0.4 to 0.6. Typically, the silica-alumina molecular sieve has an infrared absorption peak near 1450 cm⁻¹ of the pyridine adsorption infrared spectrum, which is an absorption peak of the L acid on the surface of the molecular sieve. In the pyridine adsorption infrared spectrum of the metal-modified molecular sieve of the present application, the absorption peak that originally appears near 1450 cm⁻¹ will be split into two absorption peaks in the range from 1440 cm⁻¹ to 1460 cm⁻¹, wherein the absorption peak at the lower wavenumber position is an absorption peak of the strong L acid induced by cations of the modifying metal in the channels of the molecular sieve, and the position and content of the modifying metal in the molecular sieve can be determined by calculating the proportion of the strong L acid, that is, the ratio of the area of the absorption peak at the lower wavenumber position to the total area of the two absorption peaks in the range from 1440 cm⁻¹ to 1460 cm⁻¹.

In a second aspect, a method for preparing the metal-modified molecular sieve of the present application is provided, comprising the following steps:
1) performing a first ion exchange of a molecular sieve precursor with a first exchange liquid containing a soluble compound of modifying metal;
2) subjecting the product of the first ion exchange to a first calcination to obtain a first exchanged molecular sieve;
3) performing a second ion exchange of the first exchanged molecular sieve with a second exchange liquid containing a soluble compound of modifying metal;
4) subjecting the product of the second ion exchange to a second calcination, and optionally repeating the second ion exchange and the second calcination to obtain a second exchanged molecular sieve;
5) heat-treating the second exchanged molecular sieve in an oxygen-containing atmosphere to obtain the metal-modified molecular sieve;
wherein, the first calcination and/or the second calcination is/are performed under an alkaline atmosphere.

The method for preparing the metal-modified molecular sieve provided by the present application can achieve the modification with a specific extra-framework metal through at least two exchanges and two calcinations, while ensuring the stability of the unit cell size of the molecular sieve before and after the reaction, and being able to avoid the destruction of the molecular sieve structure due to multiple calcinations.

In the present application, there is no particular limitation on the number of repeating the second ion exchange and the second calcination, which can be determined based on the effect of the ion exchange, with the aim of ensuring that the metal content in the molecular sieve meets the above range requirements, for example, it can be repeated 1-3 times.

In a preferred embodiment, the molecular sieve precursor is a silica-alumina molecular sieve, and the molar ratio of silica/alumina of the molecular sieve preferably ranges from 1:1 to 100:1, and further preferably from 3:1 to 20:1.

In the present application, the molar ratios of silica/alumina of the molecular sieve before and after metal modification remain substantially unchanged, and both meet the above-mentioned molar ratio range requirements.

In a preferred embodiment, the molecular sieve precursor is selected from at least one of X-type molecular sieve, Y-type molecular sieve, MCM-22-type molecular sieve, Beta-type molecular sieve and MOR-type molecular sieve, preferably X-type molecular sieve and/or Y-type molecular sieve.

In a preferred embodiment, the molecular sieve precursor is a hydrogen-type or sodium-type molecular sieve.

In some preferred embodiments, the molecular sieve precursor is a sodium-type molecular sieve, and the first exchange liquid and/or the second exchange liquid contain an ammonium salt; for example, the first exchange liquid contains an ammonium salt. In this case, step 1) includes ion co-exchanging the sodium-type molecular sieve with the first exchange liquid containing a soluble compound of modifying metal and an ammonium salt. In such a preferred embodiment, the use of ion co-exchanging can shorten the preparation process, and Na⁺ can be exchanged to a metal cation and NH₄⁺ in one step, and the exchanged NH₄⁺ can be decomposed and removed in the subsequent calcination process, leaving an acidic hydrogen proton for the molecular sieve.

In such a preferred embodiment, the ammonium salt may be those conventionally used in the art, and is preferably selected from ammonium nitrate, ammonium chloride, ammonium sulfate, or combinations thereof.

In a further preferred embodiment, the concentrations of the ammonium salt in the first and second exchange solutions each independently range from 50 g/L to 200 g/L, and preferably from 70 g/L to 150 g/L.

According to the present application, when the molecular sieve precursor is a sodium-type molecular sieve, in a preferred embodiment, the preparation method further comprises performing an ammonium exchange of the second exchanged molecular sieve with a third exchange liquid containing an ammonium salt, and then performing the heat treatment. In such a preferred embodiment, the Na⁺ content in the molecular sieve can be further reduced by the ammonium exchange to ensure that the molecular sieve has a higher acid amount.

In such a preferred embodiment, the ammonium exchange and drying can be carried out using conventional methods and conditions in the art, for example, the second exchanged molecular sieve is contacted with a third exchange liquid containing an ammonium salt, and then filtered and dried, which is considered as one ammonium exchange process. The ammonium exchange process can be optionally repeated 1-3 times to make the Na weight percentage in the molecular sieve less than 0.2%.

In the present application, the concentration of the ammonium salt in the third exchange solution may be in the same range as that in the first exchange liquid or the second exchange liquid, which will not be described in detail again herein.

In the present application, there is no special requirement for the specific type of the soluble compound of modifying metal, and it can be selected according to actual needs. Preferably, the soluble compound of modifying metal is selected from chloride, nitrate, phosphate, sulfate, or combinations thereof of the modifying metal.

According to the present application, there is no special requirement for the concentration of the soluble compound of modifying metal in the first exchange liquid and/or the second exchange liquid, as long as the element content of the modifying metal in the metal-modified molecular sieve meets the above range requirements, and those skilled in the art can make routine adjustments. Preferably, the concentrations of the soluble compound of modifying metal in the first exchange liquid and the second exchange liquid each independently range from 100 g/L to 500 g/L, and further preferably from 130 g/L to 400 g/L. By adopting the above preferred embodiment, the preparation process can be shortened on the basis of meeting the above content requirements.

In a preferred embodiment, the first exchange liquid and the second exchange liquid each independently also comprise a solvent, and the solvent is preferably water.

In a preferred embodiment, in order to make the first ion exchange more sufficient, the weight ratio of the first exchange liquid to the molecular sieve precursor ranges from 2:1 to 8:1, and further preferably from 3:1 to 6:1.

In a preferred embodiment, the weight ratio of the second exchange liquid to the first exchanged molecular sieve ranges from 3:1 to 6:1, and further preferably 3:1 to 5:1.

In a preferred embodiment, the conditions of the first ion exchange include: the exchange temperature ranges from 50 °C to 90 °C, and preferably from 70 °C to 90 °C; the exchange time ranges from 0.5 h to 2 h, and preferably from 0.8 h to 1.5 h.

In the present application, the conditions of the second ion exchange may be identical to or different from those of the first ion exchange. Preferably, the conditions of the second ion exchange include: the exchange temperature ranges from 50 °C to 120 °C, and preferably from 70 °C to 100 °C; the exchange time ranges from 0.5 h to 2 h, and preferably from 0.8 h to 1.5 h. Further preferably, the exchange temperature of the second ion exchange is 0-15°C, and preferably 1-10°C higher than the exchange temperature of the first exchange. The use of the above preferred embodiment is conducive to optimizing the metal distribution and further improving the catalytic activity of the molecular sieve.

In the method of the present application, the modifying metal is used in such an amount that in the obtained metal-modified molecular sieve, based on the total amount of the molecular sieve, the content of the modifying metal as element ranges from 8 wt.% to 30 wt.%, and preferably from 12 wt.% to 23 wt.%. For example, the content of the modifying metal as element can be 12 wt.%, 13 wt.%, 14 wt.%, 15 wt.%, 16 wt.%, 17 wt.%, 18 wt.%, 19 wt.%, 20 wt.%, 21 wt.%, 22 wt.%, 23 wt.% or any numerical value within a numerical range formed by any two points.

In the present application, in order to further regulate the distribution of metals, the first calcination and/or the second calcination are performed in an alkaline atmosphere, preferably, the first calcination and the second calcination are both performed in an alkaline atmosphere. The use of the above preferred embodiment is conducive to protecting the molecular sieve structure and further improving the catalytic cycle life of the metal-modified molecular sieve.

In a preferred embodiment, the alkaline atmosphere is provided by an aqueous solution of an alkaline compound. It can be understood that the aqueous solution of the alkaline compound vaporizes rapidly at the calcination temperature, thereby providing an alkaline atmosphere in the calcination process.

In the present application, the alkaline compound can be selected in a relatively wide range. Preferably, the alkaline compound is selected from ammonia, ammonium carbonate, urea, or combinations thereof, preferably ammonia.

In a further preferable embodiment, the alkaline compound is present in a concentration ranging from 0.01 mol/L to 2 mol/L, and preferably from 0.01 mol/L to 0.5 mol/L, and further preferably from 0.05 mol/L to 0.3 mol/L in the aqueous solution of the alkaline compound. In the above preferred case, it is conducive to stabilizing the structure of the molecular sieve, and prolonging the catalytic cycle life of the molecular sieve.

In a preferred embodiment, relative to 50 g of the molecular sieve precursor, the introduction rates of the alkaline atmosphere during the first and second calcinations, calculated on the volume of the aqueous solution of the alkaline compound, each independently ranges from 0.01 mL/min to 0.5 mL/min, and further preferably from 0.05 mL/min to 0.2 mL/min. It can be understood that when the amount of the molecular sieve precursor to be treated increases, the introduction rate of the alkaline atmosphere can be increased proportionally.

In a preferred embodiment, the conditions of the first calcination include: the calcination temperature ranges from 400 °C to 600 °C, the calcination time ranges from 0.5 h to 3 h, the pressure ranges from 0.01 MPa to 0.1 MPa (gauge pressure).

In a preferred embodiment, the conditions of the second calcination include: the calcination temperature ranges from 400 °C to 600 °C, the calcination time ranges from 0.5 h to 3 h, the pressure ranges from 0.01 MPa to 0.1 MPa (gauge pressure).

In the present application, the first calcination and the second calcination do not refer to the operation sequence, but are only used to distinguish the calcination conditions in different steps. The conditions of the first calcination and the second calcination can be identical or different, as long as the above conditions are met.

In a specific embodiment of the present application, before the first calcination or the second calcination, the product of the first ion exchange or the product of the second ion exchange is respectively subjected to solid-liquid separation and drying. The solid-liquid separation and drying can be performed by conventional operations in the art and will not be described in detail again herein.

In a preferred embodiment, the oxygen-containing atmosphere in step 5) is an air or a mixture of an oxygen gas and an inert gas; the inert gas is preferably nitrogen gas.

In a preferred embodiment, the oxygen gas is present in an amount ranging from 16 vol.% to 30 vol.%, and preferably from 18 vol.% to 25 vol.% in the oxygen-containing atmosphere.

In a preferred embodiment, the temperature of the heat treatment in step 5) is 50-100°C higher than the temperature of the second calcination in step 4). The heat treatment in an oxygen-containing atmosphere at a higher temperature allows the further migration of the modifying metal and the anchoring under the high temperature condition to ensure that the metal remains stable in a high temperature environment of the repetitious reaction-regeneration.

In a preferred embodiment, the conditions of the heat treatment include: the temperature ranges from 500 °C to 700 °C, and preferably from 550 °C to 650 °C, the treatment time range from 2 h to 12 h, and preferably from 4 h to 8 h.

In a third aspect, a metal-modified molecular sieve prepared by the method of the present application is provided.

In a fourth aspect, a solid acid catalyst is provided, which comprises a metal-modified molecular sieve and a heat-resistant inorganic oxide, wherein said metal-modified molecular sieve is the metal-modified molecular sieve according to the present application.

In the present application, the metal-modified molecular sieve is used as an active component in the solid acid catalyst. After extensive research, the inventors of the present application have found that when the solid acid catalyst is used in the alkylation reaction of a long-chain olefin and an aromatic hydrocarbon, the product linearity can be further improved by the coordination of the metal-modified molecular sieve of the present application and the heat-resistant inorganic oxide; simultaneously, its catalytic cycle life is also greatly extended, making it suitable for industrial production.

In the present application, for the content of each component in the solid acid catalyst, it is calculated according to the feeding ratio, and for the content of each component in the solid acid catalyst, it can be selected in a relatively wide range and can be adjusted according to actual application needs. Preferably, the weight ratio on the dry basis of the metal-modified molecular sieve to the heat-resistant inorganic oxide ranges from 99:1 to 20:80, preferably from 95:5 to 25:75, and more preferably from 90:10 to 50:50.

In the present application, there is no special requirement for the selection of the heat-resistant inorganic oxide, and specific substances conventional in the art can be used. Preferably, the heat-resistant inorganic oxide is selected from alumina, zirconia, silica, titania, clay, kaolin, montmorillonite, magnesia-alumina spinel, amorphous silica-alumina, or combinations thereof, more preferably selected from alumina, zirconia, silica, titania, or combinations thereof. The use of the above-mentioned preferred heat-resistant inorganic oxide is conducive to the coordination of the metal-modified molecular sieve and the heat-resistant inorganic oxide, and further improving the catalytic performance of the catalyst.

In a preferred embodiment, the heat-resistant inorganic oxide has a particle size ranging 0.005 µm to 200 µm, and further preferably from 0.01 µm to 100 µm. In the above preferred case, it is conductive to enhancing the interaction between the molecular sieve and the inorganic oxide, and improving the performance and strength of the catalyst.

In a fifth aspect, a method for preparing the solid acid catalyst of the present application is provided, comprising: mixing the metal-modified molecular sieve of the present application, a heat-resistant inorganic oxide and/or a precursor thereof, water, an optional extrusion aid, and an optional peptizing agent, molding and calcining to produce the solid acid catalyst.

In the present application, the precursor of the heat-resistant inorganic oxide is a substance that can be calcined to produce a heat-resistant inorganic oxide, which is a conventional choice in the art and to which the present application has no special requirements. For example, the precursor of alumina can be pseudo-boehmite.

Preferably, the heat-resistant inorganic oxide and/or a precursor thereof has a particle size ranging from 0.005 µm to 200 µm, and further preferably 0.01 µm to 100 µm. In the above preferred case, it is conductive to enhancing the interaction between the molecular sieve and the inorganic oxide, and improving the performance and strength of the catalyst.

According to the present application, an extrusion aid and/or a peptizing agent may or may not be added during the mixing process, and a selection may be made according to actual needs in molding.

In the present application, there is no particular limitation on the type of the extrusion aid, and conventional extrusion aids in the art are all applicable to the present application. Preferably, the extrusion aid is selected from sesbania powder, cellulose, starch, or combinations thereof.

In the present application, the amount of the extrusion aid used can be selected in a relatively wide range. Preferably, the extrusion aid is used in an amount ranging from 0 wt.% to 20 wt.% based on the total weight of the metal-modified molecular sieve and the heat-resistant inorganic oxide and/or a precursor thereof on a dry basis, further preferably, the extrusion aid is used in an amount ranging from 1 wt.% to 5 wt.% based on the total weight of the metal-modified molecular sieve and the heat-resistant inorganic oxide on a dry basis.

In the present application, there is no particular limitation on the type of the peptizing agent, and conventional peptizing agents in the art are all applicable to the present application. Preferably, the peptizing agent is selected from citric acid, nitric acid, phosphoric acid, or combinations thereof. In the present application, there is no particular limitation on the amount of the peptizing agent used. Preferably, the peptizing agent is used in an amount ranging from 0 wt.% to 20wt.% based on the total weight of the metal-modified molecular sieve and the heat-resistant inorganic oxide and/or a precursor thereof on a dry basis, further preferably, the peptizing agent is used in an amount ranging from 1 wt.% to 8 wt.% based on the total weight of the metal-modified molecular sieve and the heat-resistant inorganic oxide and/or a precursor thereof on a dry basis.

In the present application, water can be used in a relatively wide range of amounts, as long as the above components can be mixed evenly, and can be adjusted according to actual needs. Preferably, the ratio of the weight of the water used to the total weight of the metal-modified molecular sieve and the heat-resistant inorganic oxide and/or a precursor thereof on a dry basis ranges from 0.2:1 to 1.2:1, further preferably, the ratio of the weight of the water used to the total weight of the metal-modified molecular sieve and the heat-resistant inorganic oxide and/or a precursor thereof on a dry basis ranges from 0.3:1 to 1:1.

In the present application, as required, the method further comprises drying the shaped product or molding-drying to obtain a mixed shaped product, and then performing the calcination. The drying or molding-drying can be performed by conventional operations in the art, such as drying or spray-drying, so that the weight on the dry basis of the mixed shaped product ranges from 40 wt.% to 85 wt.%, and preferably from 60 wt.% to 75 wt.% of the weight of the shaped product.

In a preferred embodiment, the temperature of drying or molding-drying ranges from 100 °C to 250 °C, and preferably from 100 °C to 150 °C, the time of drying or molding-drying ranges from 1 h to 24 h, and preferably from 2 h to 10 h.

In a preferred embodiment, the temperature for calcining once ranges from 400 °C to 650 °C, preferably from 500 °C to 600 °C, and the time for calcining once ranges from 0.5 h to 8 h, and preferably from 2 h to 6 h.

In a sixth aspect, a solid acid catalyst prepared by the method of the present application is provided.

In a seventh aspect, the use of the metal-modified molecular sieve or the solid acid catalyst of the present application in the preparation of a long-chain hydrocarbon-based aromatic compound is provided, wherein the preparation of the long-chain hydrocarbon-based aromatic compound is achieved by the reaction of a long-chain olefin and an aromatic hydrocarbon.

In an eighth aspect, the present application provides a method for preparing a long-chain hydrocarbon-based aromatic compound, comprising a step of reacting a long-chain olefin and an aromatic hydrocarbon in the presence of the metal-modified molecular sieve of the present application.

In the method for preparing the long-chain hydrocarbon-based aromatic compound of the present application, due to the shape selectivity on the reaction product after the modification of the molecular sieve, the product linearity is significantly improved, and the product selectivity is improved. The improvement in the product selectivity also significantly extends the catalytic cycle life of the metal-modified molecular sieve.

In the present application, there is no special requirement for the source of the long-chain olefin, for example, the long-chain olefins obtained by dehydrogenation and cracking of paraffin hydrocarbons and oligomerization of small molecule olefins or those commercially available can be used in the present application. Preferably, the long-chain olefin is a long-chain alpha olefin having a double bond at the end. Preferably, the long-chain olefin is a C7-C27 long-chain olefin, further preferably a C8-C15 long-chain olefin, and more preferably a C10-C14 long-chain olefin.

According to the present application, the aromatic hydrocarbon can be a monocyclic or bicyclic aromatic hydrocarbon, preferably a benzene-based aromatic hydrocarbon, more preferably selected from benzene, toluene, ethylbenzene, or combinations thereof, particularly preferably benzene.

In the present application, the reaction of the long-chain olefin and the aromatic hydrocarbon can be carried out under conventional conditions in the art, preferably ensuring that the reaction is carried out in the liquid phase and the pressure is always sufficient to ensure that the reaction is carried out in a single liquid phase. In the present application, the single liquid phase means that the fed reactants are all in the liquid phase, and the reaction conditions ensure that the reactants will not vaporize during the reaction. Preferably, the reaction pressure is higher than the saturated vapor pressure of the long-chain olefin and the aromatic hydrocarbon at the corresponding reaction temperature, so that the reaction materials are all in the liquid phase to perform the contacting and reacting. In a preferred embodiment, the reaction condition includes: the reaction temperature ranges from 50 °C to 250 °C, and preferably from 70 °C to 200°C; the reaction pressure ranges from 0.1 MPa to 7 MPa, and preferably from 2 MPa to 4 MPa; the molar ratio of aromatic hydrocarbon/olefin ranges from 3:1 to 70:1, and preferably from 4:1 to 60:1; the weight space velocity of the long-chain olefin ranges from 0.1 h⁻¹ to 5 h⁻¹, and preferably from 0.3 h⁻¹ to 3 h⁻¹. The use of the preferred reaction conditions described above contributes to reducing isomerization of the alkyl groups and minimizing polyalkylation of benzene (or the aromatic moiety of other aryl compounds), while maximizing the consumption of olefin to maximize the product.

According to the present application, the reaction of the long-chain olefin and the aromatic hydrocarbon can be carried out in a conventional reactor in the art. For example, the reaction can be carried out in a fixed bed reactor, a fluidized bed reactor, a moving bed reactor or a slurry bed reactor.

In some preferred embodiments, the reaction of the long-chain olefin and the aromatic hydrocarbon is carried out in the presence of the solid acid catalyst of the present application, and the method further comprises a step of regenerating the solid acid catalyst under an oxygen-containing mixed gas. In such preferred embodiments, the catalyst can be regenerated conveniently by regeneration, and a long-term continuous operation can be achieved by reaction-regeneration.

In a further preferred embodiment, the oxygen gas is present in an amount ranging from 1 vol.% to 50 vol.%, and preferably 10 vol.% to 25 vol.% in the oxygen-containing mixed gas.

In a further preferred embodiment, the oxygen-containing mixed gas is a mixture of an oxygen gas and a protective gas; the protective gas can be any gas that does not participate in the reaction, and preferably, the protective gas is at least one of nitrogen gas, helium gas, and argon gas.

In a further preferred embodiment, the regeneration temperature ranges from 400 °C to 700 °C, the regeneration time ranges from 4 h to 18 h, the volume space velocity of the oxygen-containing mixed gas ranges from 500 h⁻¹ to 6000 h⁻¹, and the regeneration pressure ranges from 0.01 MPa to 0.1 MPa (gauge pressure).

In still further preferred embodiment, the regeneration temperature ranges from 450 °C to 600 °C, e.g. it can be 450 °C, 460 °C, 470 °C, 480 °C, 490 °C, 500 °C, 510 °C, 520 °C, 530 °C, 540 °C, 550 °C, 560 °C, 570 °C, 580 °C, 590 °C, 600 °C or the like, which is a typical but not limiting temperature; preferably, the regeneration time ranges from 5 h to 12 h, the volume space velocity of the oxygen-containing mixed gas ranges from 500 h⁻¹ to 5000 h⁻¹, the regeneration pressure ranges from 0.01 MPa to 0.05 MPa (gauge pressure). The use of the above preferred regeneration conditions is conducive to improving the regeneration effect of the solid acid catalyst.

According to the present application, by periodically regenerating the solid acid catalyst, a long-term operation of the reaction can be achieved. The timing of the regeneration is selected by detecting the progress of the reaction, for example, the regeneration is performed when the long-chain olefin conversion rate is less than 95%, and further preferably, the regeneration is performed when the long-chain olefin conversion rate is less than 98%.

In some preferred embodiments, the present application provides the following technical solutions:
A1. A metal-modified molecular sieve, characterized in that the metal-modified molecular sieve comprises a molecular sieve and an extra-framework metal of the molecular sieve; based on the total amount of the metal-modified molecular sieve, the content of the modifying metal as element ranges from 8 wt.% to 30 wt.%;
   wherein, in the metal-modified molecular sieve, the ratio of the weight percentage of the surface-layer modifying metal measured by XPS to the weight percentage of the bulk modifying metal measured by XRF does not exceed 1.45, calculated on the basis of the weight percentage of the element.
A2. The metal-modified molecular sieve according to Item A1, wherein in the metal-modified molecular sieve, the ratio of the weight percentage of the surface-layer modifying metal measured by XPS to the weight percentage of the bulk modifying metal measured by XRF does not exceed 1.35, preferably ranges from 0.9 to 1.35, calculated on the basis of the weight percentage of the element;
   preferably, based on the total amount of the metal-modified molecular sieve, the content of the modifying metal as element ranges from 12 wt.% to 23 wt.%;
   preferably, the modifying metal has an atomic radius ranging from 120 pm to 270 pm, and preferably from 160 pm to 240 pm;
   preferably, the modifying metal is selected from at least one of an alkaline earth metal, a Group IIIB metal and a Group IIIA metal; further preferably at least one of La, Ce, Sr and Y.
A3. The metal-modified molecular sieve according to Item A1 or A2, wherein the metal-modified molecular sieve is a silica-alumina molecular sieve;
   preferably, the molar ratio of silica/alumina in the metal-modified molecular sieve ranges from 1:1 to 100:1, and preferably from 3:1 to 20:1;
   preferably, the metal-modified molecular sieve is selected from at least one of X-type molecular sieve, Y-type molecular sieve, MCM-22-type molecular sieve, Beta-type molecular sieve and MOR-type molecular sieve, preferably X-type molecular sieve and/or Y-type molecular sieve.
A4. A method for preparing a metal-modified molecular sieve, comprising the following steps:
   1) performing a first ion exchange of a molecular sieve precursor with a first exchange liquid containing a soluble compound of modifying metal;
   2) subjecting the product of the first ion exchange to a first calcination to obtain a first exchanged molecular sieve;
   3) performing a second ion exchange of the first exchanged molecular sieve with a second exchange liquid containing a soluble compound of modifying metal;
   4) subjecting the product of the second ion exchange to a second calcination, and optionally repeating the second ion exchange and the second calcination to obtain a second exchanged molecular sieve;
   5) heat-treating the second exchanged molecular sieve in an oxygen-containing atmosphere to obtain the metal-modified molecular sieve;

   wherein, the first calcination and/or the second calcination is/are performed under an alkaline atmosphere;
   wherein based on the total amount of the metal-modified molecular sieve, the content of the modifying metal as element ranges from 8 wt.% to 30 wt.%.
A5. The preparation method according to Item A4, wherein, based on the total amount of the metal-modified molecular sieve, the content of the modifying metal as element ranges from 12 wt.% to 23 wt.%.
A6. The preparation method according to Item A4 or A5, wherein the molecular sieve precursor is a hydrogen-type or sodium-type molecular sieve;
   preferably, the molecular sieve precursor is a silica-alumina molecular sieve, preferably the molar ratio of silica/alumina in the molecular sieve ranges from 1:1 to 100:1, and preferably 3:1 to 20:1; preferably, the molecular sieve precursor is selected from at least one of X-type molecular sieve, Y-type molecular sieve, MCM-22-type molecular sieve, Beta-type molecular sieve and MOR-type molecular sieve, preferably X-type molecular sieve and/or Y-type molecular sieve;
   preferably, the molecular sieve precursor is a sodium-type molecular sieve, the first exchange liquid and/or the second exchange liquid further comprise an ammonium salt;
   preferably, the ammonium salt is selected from ammonium nitrate, ammonium chloride, ammonium sulfate, or combinations thereof;
   preferably, the concentrations of the ammonium salt in the first exchange liquid and/or the second exchange liquid each independently range from 50 g/L to 200 g/L.
A7. The preparation method according to any one of Items A4-A6, wherein the soluble compound of modifying metal is selected from chloride, nitrate, phosphate, sulfate, or combinations thereof of the modifying metal;
   preferably, the concentrations of the soluble compound of modifying metal in the first exchange liquid and/or the second exchange liquid each independently range from 100 g/L to 500 g/L;
   preferably, the first exchange liquid and the second exchange liquid each independently also comprise a solvent, and the solvent is preferably water;
   preferably, the modifying metal has an atomic radius ranging from 120 pm to 270 pm, and preferably from 160 pm to 240 pm;
   preferably, the modifying metal is selected from at least one of an alkaline earth metal, a Group IIIB metal and a Group IIIA metal; further preferably at least one of La, Ce, Sr and Y;
   preferably, the weight ratio of the first exchange liquid to the molecular sieve precursor ranges from 2:1 to 8:1;
   preferably, the weight ratio of the second exchange liquid to the first exchanged molecular sieve ranges from 3:1 to 6:1;
   preferably, the conditions of the first ion exchange include: the exchange temperature ranges from 50 °C to 90 °C, and preferably from 70 °C to 90 °C; the exchange time ranges from 0.5 h to 2 h, and preferably from 0.5 h to 1.5 h;
   preferably, the exchange temperature of the second ion exchange is 0-15°C, and preferably 1-10°C higher than the exchange temperature of the first ion exchange.
A8. The preparation method according to any one of Items A4-A7, wherein the alkaline atmosphere is provided by an aqueous solution of the alkaline compound;
   preferably, the alkaline compound is selected from ammonia, ammonium carbonate, urea, or combinations thereof;
   preferably, the alkaline compound is present in a concentration ranging from 0.01 mol/L to 2 mol/L in the aqueous solution of the alkaline compound;
   preferably, relative to 50 g of the molecular sieve precursor, the introduction rate of the alkaline atmosphere, calculated on the volume of the aqueous solution of the alkaline compound, ranges from 0.01 mL/min to 0.5 mL/min;
   preferably, the conditions of the first calcination include: the calcination temperature ranges from 400 °C to 600 °C, the calcination time ranges from 0.5 h to 3 h, the pressure ranges from 0.01 MPa to 0.1 MPa (gauge pressure);
   preferably, the condition of the second calcination includes: the calcination temperature ranges from 400 °C to 600 °C, the calcination time ranges from 0.5 h to 3 h, the pressure ranges from 0.01 MPa to 0.1 MPa (gauge pressure).
A9. The preparation method according to any one of Items A4-A8, wherein the oxygen-containing atmosphere is an air or a mixture of an oxygen gas and an inert gas;
   preferably, the oxygen gas is present in an amount ranging from 16 vol.% to 30 vol.%, and preferably from 18 vol.% to 25 vol.% in the oxygen-containing atmosphere;
   preferably, the temperature of the heat treatment is 50-100°C higher than the temperature of the second calcination;
   preferably, the conditions of the heat treatment include: the temperature ranges from 500 °C to 700 °C, and preferably from 550 °C to 650 °C, the treatment time ranges from 2 h to 12 h, and preferably from 4 h to 8 h.
A10. A metal-modified molecular sieve prepared with the preparation method according to any one of Items A4-A9.
A11. A method for alkylating a long-chain olefin, wherein the method comprises: contacting the long-chain olefin and an aromatic hydrocarbon with a catalyst under an alkylation reaction condition to carry out an alkylation reaction;
   characterized in that, the catalyst is the metal-modified molecular sieve according to any one of Items A1-A3 and A10.
A12. The method according to Item A11, wherein the long-chain olefin is a long-chain alpha olefin having a double bond at the end;
   preferably, the long-chain olefin is a C8-C28 long-chain olefin, further preferably a C8-C15 long-chain olefin, and more preferably a C10-C14 long-chain olefin;
   preferably, the aromatic hydrocarbon is selected from benzene, toluene, ethylbenzene, or combinations thereof, preferably benzene;
   preferably, the alkylation reaction conditions include: the reaction temperature ranges from 50 °C to 250 °C, and preferably from 70 °C to 200 °C; the reaction pressure ranges from 0.1 MPa to 7 MPa, and preferably from 2 MPa to 4 MPa; the molar ratio of benzene/olefin ranges from 3:1 to 70:1, and preferably 4:1 to 60:1; the weight space velocity of the long-chain olefin ranges from 0.1 h⁻¹ to 5 h⁻¹, and preferably from 0.3 h⁻¹ to 3 h⁻¹.
B1. A solid acid catalyst, characterized in that the catalyst comprises a metal-modified molecular sieve and a heat-resistant inorganic oxide;
   wherein the metal-modified molecular sieve comprises a molecular sieve and an extra-framework metal of the molecular sieve; based on the total amount of the metal-modified molecular sieve, the content of the modifying metal as element ranges from 8 wt.% to 30 wt.%;
   wherein, in the metal-modified molecular sieve, calculated on the basis of the weight percentage of the element, the ratio of the weight percentage of the surface-layer modifying metal measured by XPS to the weight percentage of the bulk modifying metal measured by XRF does not exceed 1.45.
B2. The solid acid catalyst according to Item B1, wherein the weight ratio of the metal-modified molecular sieve to the heat-resistant inorganic oxide ranges from 99:1 to 20:80, preferably from 95:5 to 25:75, and more preferably from 90:10 to 50:50 on a dry basis.
B3. The solid acid catalyst according to Item B1 or B2, wherein the heat-resistant inorganic oxide is selected from alumina, zirconia, silica, titania, or combinations thereof;
   preferably, the heat-resistant inorganic oxide has a particle size ranging from 0.005 µm to 200 µm, and further preferably from 0.01 µm to 100 µm.
B4. The solid acid catalyst according to any one of Items B1-B3, wherein in the metal-modified molecular sieve, the ratio of the weight percentage of the surface-layer modifying metal measured by XPS to the weight percentage of the bulk modifying metal measured by XRF does not exceed 1.35, and preferably ranges from 0.9 to 1.35, calculated on the basis of the weight percentage of the element;
   preferably, based on the total amount of the metal-modified molecular sieve, the content of the modifying metal as element ranges from 12 wt.% to 23 wt.%;
   preferably, the modifying metal has an atomic radius ranging from 120 pm to 270 pm, and preferably from 160 pm to 240 pm;
   preferably, the modifying metal is selected from at least one of an alkaline earth metal, a Group IIIB metal and a Group IIIA metal; further preferably at least one of La, Ce, Sr and Y.
B5. The solid acid catalyst according to any one of Items B1-B4, wherein the metal-modified molecular sieve is a silica-alumina molecular sieve;
   preferably, the molar ratio of silica/alumina in the metal-modified molecular sieve ranges from 1:1 to 100:1, and preferably from 3:1 to 20:1;
   preferably, the metal-modified molecular sieve is selected from at least one of X-type molecular sieve, Y-type molecular sieve, MCM-22-type molecular sieve, Beta-type molecular sieve and MOR-type molecular sieve, preferably X-type molecular sieve and/or Y-type molecular sieve.
B6. A method for preparing a solid acid catalyst, characterized in that the preparation method comprises: mixing a metal-modified molecular sieve, a heat-resistant inorganic oxide and/or a precursor thereof, water, an optional extrusion aid, and an optional peptizing agent, molding and calcining to produce the solid acid catalyst;
   wherein the metal-modified molecular sieve comprises a molecular sieve and an extra-framework metal of the molecular sieve; based on the total amount of the metal-modified molecular sieve, the content of the modifying metal as element ranges from 8 wt.% to 30 wt.%;
   wherein, in the metal-modified molecular sieve, calculated on the basis of the weight percentage of the element, the ratio of the weight percentage of the surface-layer modifying metal measured by XPS to the weight percentage of the bulk modifying metal measured by XRF does not exceed 1.45.
B7. The preparation method according to Item B6, wherein the metal-modified molecular sieve is prepared by the following method, which comprises:
   1) performing a first ion exchange of a molecular sieve precursor with a first exchange liquid containing a soluble compound of modifying metal;
   2) subjecting the product of the first ion exchange to a first calcination to obtain a first exchanged molecular sieve;
   3) performing a second ion exchange of the first exchanged molecular sieve with a second exchange liquid containing a soluble compound of modifying metal;
   4) subjecting the product of the second ion exchange to a second calcination, and optionally repeating the second ion exchange and the second calcination to obtain a second exchanged molecular sieve;
   5) heat-treating the second exchanged molecular sieve in an oxygen-containing atmosphere to obtain the metal-modified molecular sieve;
   wherein, the first calcination and/or the second calcination is/are performed under an alkaline atmosphere.
B8. The preparation method according to Item B7, wherein the molecular sieve precursor is a hydrogen-type or sodium-type molecular sieve;
   preferably, the molecular sieve precursor is a silica-alumina molecular sieve, preferably the molar ratio of silica/alumina in the molecular sieve ranges from 1:1 to 100:1, and preferably 3:1 to 20:1; preferably, the molecular sieve precursor is selected from at least one of X-type molecular sieve, Y-type molecular sieve, MCM-22-type molecular sieve, Beta-type molecular sieve and MOR-type molecular sieve, preferably X-type molecular sieve and/or Y-type molecular sieve;
   preferably, the molecular sieve precursor is a sodium-type molecular sieve, the first exchange liquid and/or the second exchange liquid further comprise an ammonium salt;
   preferably, the ammonium salt is selected from ammonium nitrate, ammonium chloride, ammonium sulfate, or combinations thereof;
   preferably, the concentrations of the ammonium salt in the first exchange liquid and/or the second exchange liquid each independently range from 50 g/L to 200 g/L.
B9. The preparation method according to Item B7 or B8, wherein the soluble compound of modifying metal is selected from chloride, nitrate, phosphate, sulfate, or combinations thereof of the modifying metal;
   preferably, the first exchange liquid and the second exchange liquid each independently also comprise a solvent, and the solvent is preferably water;
   preferably, the concentrations of the soluble compound of modifying metal in the first exchange liquid and/or the second exchange liquid each independently range from 100 g/L to 500 g/L;
   preferably, the modifying metal has an atomic radius ranging from 120 pm to 270 pm, and preferably from 160 pm to 240 pm;
   preferably, the modifying metal is selected from at least one of an alkaline earth metal, a Group IIIB metal and a Group IIIA metal; further preferably at least one of La, Ce, Sr and Y;
   preferably, the weight ratio of the first exchange liquid to the molecular sieve precursor ranges from 2:1 to 8:1;
   preferably, the weight ratio of the second exchange liquid to the first exchanged molecular sieve ranges from 3:1 to 6:1;
   preferably, the conditions of the first ion exchange include: the exchange temperature ranges from 50 °C to 90 °C, and preferably from 70 °C to 90 °C; the exchange time ranges from 0.5 h to 2 h, and preferably from 0.5 h to 1.5 h;
   preferably, the exchange temperature of the second ion exchange is 0-15°C, and preferably 1-10°C higher than the exchange temperature of the first ion exchange;
   preferably, based on the total amount of the metal-modified molecular sieve, the content of the modifying metal as element ranges from 12 wt.% to 23 wt.%.
B10. The preparation method according to any one of Items B7-B9, wherein the alkaline atmosphere is provided by an aqueous solution of the alkaline compound;
   preferably, the alkaline compound is selected from ammonia, ammonium carbonate, urea, or combinations thereof;
   preferably, the alkaline compound is present in a concentration ranging from 0.01 mol/L to 2 mol/L in the aqueous solution of the alkaline compound;
   preferably, relative to 50 g of the molecular sieve precursor, the introduction rate of the alkaline atmosphere, calculated on the volume of the aqueous solution of the alkaline compound, ranges from 0.01 mL/min to 0.5 mL/min;
   preferably, the conditions of the first calcination include: the calcination temperature ranges from 400 °C to 600 °C, the calcination time ranges from 0.5 h to 3 h, the pressure ranges from 0.01 MPa to 0.1 MPa (gauge pressure);
   preferably, the conditions of the second calcination include: the calcination temperature ranges from 400 °C to 600 °C, the calcination time ranges from 0.5 h to 3 h, the pressure ranges from 0.01 MPa to 0.1 MPa (gauge pressure).
B11. The preparation method according to any one of Items B7-B10, wherein the oxygen-containing atmosphere is an air or a mixture of an oxygen gas and an inert gas;
   preferably, the oxygen gas is present in an amount ranging from 16 vol.% to 30 vol.%, and preferably from 18 vol.% to 25 vol.% in the oxygen-containing atmosphere;
   preferably, the temperature of the heat treatment is 50-100°C higher than the temperature of the second calcination;
   preferably, the conditions of the heat treatment include: the temperature ranges from 500 °C to 700 °C, and preferably from 550 °C to 650 °C, the treatment time ranges from 2 h to 12 h, and preferably from 4 h to 8 h.
B12. The preparation method according to any one of Items B6-B11, wherein the weight ratio of the metal-modified molecular sieve to the heat-resistant inorganic oxide and/or a precursor thereof ranges from 99:1 to 20:80, preferably from 95:5 to 25:75, and more preferably from 90:10 to 50:50 on a dry basis.
B13. The preparation method according to any one of Items B6-B12, wherein the heat-resistant inorganic oxide is selected from alumina, zirconia, silica, titania, or combinations thereof;
   preferably, the heat-resistant inorganic oxide and/or a precursor thereof has a particle size ranging from 0.005 µm to 200 µm, and further preferably from 0.01 µm to 100 µm;
   preferably, the extrusion aid is selected from sesbania powder, cellulose, starch, or combinations thereof;
   preferably, the extrusion aid is used in an amount ranging from 0 wt.% to 20 wt.% based on the total weight of the metal-modified molecular sieve and the heat-resistant inorganic oxide and/or a precursor thereof on a dry basis, further preferably, the extrusion aid is used in an amount ranging from 1 wt.% to 5 wt.% based on the total weight of the metal-modified molecular sieve and the heat-resistant inorganic oxide and/or a precursor thereof on a dry basis;
   preferably, the peptizing agent is selected from citric acid, nitric acid, phosphoric acid, or combinations thereof;
   preferably, the peptizing agent is used in an amount ranging from 0 wt.% to 20 wt.% based on the total weight of the metal-modified molecular sieve and the heat-resistant inorganic oxide and/or a precursor thereof on a dry basis, further preferably, the peptizing agent is used in an amount ranging from 1 wt.% to 8 wt.% based on the total weight of the metal-modified molecular sieve and the heat-resistant inorganic oxide and/or a precursor thereof on a dry basis;
   preferably, the ratio of the weight of the water used to the total weight of the metal-modified molecular sieve and the heat-resistant inorganic oxide and/or a precursor thereof on a dry basis ranges from 0.2:1 to 1.2:1, further preferably, the ratio of the weight of the water used to the total weight of the metal-modified molecular sieve and the heat-resistant inorganic oxide and/or a precursor thereof on a dry basis ranges from 0.3:1 to 1:1;
   preferably, the calcining temperature ranges from 400 °C to 650 °C, the calcining time ranges from 1 h to 12 h.
B14. A solid acid catalyst prepared with the preparation method according to any one of Items B6-B13.
B15. Use of the solid acid catalyst according to any one of Items B1-B5 and B14 in the preparation of long-chain alkylbenzenes.
C1. A method for preparing a long-chain alkylbenzene, characterized in that the method comprises: contacting a mixture of a long-chain olefin and an aromatic hydrocarbon with a solid acid catalyst under an alkylation reaction condition to obtain the long-chain alkylbenzene;
   the method further optionally includes: regenerating the solid acid catalyst under an oxygen-containing mixed gas;
   wherein, the solid acid catalyst comprises a metal-modified molecular sieve and a heat-resistant inorganic oxide;
   wherein the metal-modified molecular sieve comprises a molecular sieve and an extra-framework metal of the molecular sieve; based on the total amount of the metal-modified molecular sieve, the content of the modifying metal as element ranges from 8 wt.% to 30 wt.%; the ratio of the weight percentage of the surface-layer modifying metal measured by XPS to the weight percentage of the bulk modifying metal measured by XRF does not exceed 1.45, calculated on the basis of the weight percentage of the element.
C2. The preparation method according to Item C1, wherein based on the total amount of the solid acid catalyst, the weight ratio of the metal-modified molecular sieve to the heat-resistant inorganic oxide ranges from 99:1 to 20:80, preferably from 95:5 to 25:75, and more preferably from 90:10 to 50:50 on a dry basis.
C3. The preparation method according to Item C1 or C2, wherein the heat-resistant inorganic oxide is selected from alumina, zirconia, silica, titania, or combinations thereof;
   preferably, the heat-resistant inorganic oxide has a particle size ranging from 0.005 µm to 200 µm, and further preferably from 0.01 µm to 100 µm.
C4. The preparation method according to any one of Items C1-C3, wherein in the metal-modified molecular sieve, the ratio of the weight percentage of the surface-layer modifying metal measured by XPS to the weight percentage of the bulk modifying metal measured by XRF does not exceed 1.35, preferably ranges from 0.9 to 1.35, calculated on the basis of the weight percentage of the element;
   preferably, based on the total amount of the metal-modified molecular sieve, the content of the modifying metal as element ranges from 12 wt.% to 23 wt.%;
   preferably, the modifying metal has an atomic radius ranging from 120 pm to 270 pm, and preferably from 160 pm to 240 pm;
   preferably, the modifying metal is selected from at least one of an alkaline earth metal, a Group IIIB metal and a Group IIIA metal; further preferably at least one of La, Ce, Sr and Y.
C5. The preparation method according to any one of Items C1-C4, wherein the metal-modified molecular sieve is a silica-alumina molecular sieve;
   preferably, the molar ratio of silica/alumina in the metal-modified molecular sieve ranges from 1:1 to 100:1, and preferably from 3:1 to 20:1;
   preferably, the metal-modified molecular sieve is selected from at least one of X-type molecular sieve, Y-type molecular sieve, MCM-22-type molecular sieve, Beta-type molecular sieve and MOR-type molecular sieve, preferably X-type molecular sieve and/or Y-type molecular sieve.
C6. The preparation method according to any one of Items C1-C5, wherein the preparation method of the solid acid catalyst comprises: mixing a metal-modified molecular sieve, a heat-resistant inorganic oxide and/or a precursor thereof, water, an optional extrusion aid, and an optional peptizing agent, molding and calcining to produce the solid acid catalyst.
C7. The preparation method according to any one of Items C1-C6, wherein the metal-modified molecular sieve is prepared by the following method, which comprises:
   1) performing a first ion exchange of a molecular sieve precursor with a first exchange liquid containing a soluble compound of modifying metal;
   2) subjecting the product of the first ion exchange to a first calcination to obtain a first exchanged molecular sieve;
   3) performing a second ion exchange of the first exchanged molecular sieve with a second exchange liquid containing a soluble compound of modifying metal;
   4) subjecting the product of the second ion exchange to a second calcination, and optionally repeating the second ion exchange and the second calcination to obtain a second exchanged molecular sieve;
   5) heat-treating the second exchanged molecular sieve in an oxygen-containing atmosphere to obtain the metal-modified molecular sieve;
   wherein, the first calcination and/or the second calcination is/are performed under an alkaline atmosphere.
C8. The preparation method according to Item C7, wherein the molecular sieve precursor is a hydrogen-type or sodium-type molecular sieve;
   preferably, the molecular sieve precursor is a silica-alumina molecular sieve, preferably the molar ratio of silica/alumina in the molecular sieve ranges from 1:1 to 100:1, and preferably from 3:1 to 20:1;
   preferably, the molecular sieve precursor is selected from at least one of X-type molecular sieve, Y-type molecular sieve, MCM-22-type molecular sieve, Beta-type molecular sieve and MOR-type molecular sieve, preferably X-type molecular sieve and/or Y-type molecular sieve;
   preferably, the molecular sieve precursor is a sodium-type molecular sieve, the first exchange liquid and/or the second exchange liquid further comprise an ammonium salt;
   preferably, the ammonium salt is selected from ammonium nitrate, ammonium chloride, ammonium sulfate, or combinations thereof;
   preferably, the concentrations of the ammonium salt in the first exchange liquid and/or the second exchange liquid each independently range from 50 g/L to 200 g/L.
C9. The preparation method according to Item C7 or C8, wherein the soluble compound of modifying metal is selected from chloride, nitrate, phosphate, sulfate, or combinations thereof of the modifying metal;
   preferably, the first exchange liquid and the second exchange liquid each independently also comprise a solvent, and the solvent is preferably water;
   preferably, the concentrations of the soluble compound of modifying metal in the first exchange liquid and/or the second exchange liquid each independently range from 100 g/L to 500 g/L;
   preferably, the weight ratio of the first exchange liquid to the molecular sieve precursor ranges from 2:1 to 8:1;
   preferably, the weight ratio of the second exchange liquid to the first exchanged molecular sieve ranges from 3:1 to 6:1;
   preferably, the conditions of the first ion exchange include: the exchange temperature ranges from 50 °C to 90 °C, and preferably from 70 °C to 90 °C; the exchange time ranges from 0.5 h to 2 h, and preferably from 0.5 h to 1.5 h;
   preferably, the exchange temperature of the second ion exchange is 0-15°C, and preferably 1-10°C higher than the exchange temperature of the first ion exchange.
C10. The preparation method according to any one of Items C7-C9, wherein the alkaline atmosphere is provided by an aqueous solution of the alkaline compound;
   preferably, the alkaline compound is selected from ammonia, ammonium carbonate, urea, or combinations thereof;
   preferably, the alkaline compound is present in a concentration ranging from 0.01 mol/L to 2 mol/L in the aqueous solution of the alkaline compound;
   preferably, relative to 50 g of the molecular sieve precursor, the introduction rate of the alkaline atmosphere, calculated on the volume of the aqueous solution of the alkaline compound, ranges from 0.01 mL/min to 0.5 mL/min;
   preferably, the conditions of the first calcination include: the calcination temperature ranges from 400 °C to 600 °C, the calcination time ranges from 0.5 h to 3 h, the pressure ranges from 0.01 MPa to 0.1 MPa (gauge pressure);
   preferably, the conditions of the second calcination include: the calcination temperature ranges from 400 °C to 600 °C, the calcination time ranges from 0.5 h to 3 h, the pressure ranges from 0.01 MPa to 0.1 MPa (gauge pressure).
C11. The preparation method according to any one of Items C7-C10, wherein the oxygen-containing atmosphere is an air or a mixture of an oxygen gas and an inert gas;
   preferably, the oxygen gas is present in an amount ranging from 16 vol.% to 30 vol.%, and preferably from 18 vol.% to 25 vol.% in the oxygen-containing atmosphere;
   preferably, the temperature of the heat treatment is 50-100°C higher than the temperature of the second calcination;
   preferably, the conditions of the heat treatment include: the temperature ranges from 500 °C to 700 °C, and preferably from 550 °C to 650 °C, the treatment time ranges from 2 h to 12 h, and preferably from 4 h to 8 h.
C12. The preparation method according to any one of Items C6-C11, wherein the extrusion aid is selected from sesbania powder, cellulose, starch, or combinations thereof;
   preferably, the extrusion aid is used in an amount ranging from 0 wt.% to 5 wt.% based on the total weight on a dry basis of the metal-modified molecular sieve and the heat-resistant inorganic oxide; preferably, the peptizing agent is selected from citric acid, nitric acid, phosphoric acid, or combinations thereof;
   preferably, the peptizing agent is used in an amount ranging from 0 wt.% to 8 wt.% based on the total weight on a dry basis of the metal-modified molecular sieve and the heat-resistant inorganic oxide;
   preferably, the ratio of the weight of the water used to the total weight on a dry basis of the metal-modified molecular sieve and the heat-resistant inorganic oxide ranges from 0.1:1 to 1:1;
   preferably, the calcining temperature ranges from 400 °C to 700 °C, the calcining time ranges from 2 h to 12 h.
C13. The preparation method according to any one of Items C1-C12, wherein the long-chain olefin is a long-chain alpha olefin having a double bond at the end;
   preferably, the long-chain olefin is selected from C7-C27 long-chain olefin;
   preferably, the aromatic hydrocarbon is selected from benzene, toluene, ethylbenzene, or combinations thereof, preferably benzene;
   preferably, the alkylation reaction conditions include: the reaction temperature ranges from 50 °C to 250 °C; the pressure ranges from 0.1 MPa to 7 MPa; the molar ratio of benzene/olefin ranges from 3:1 to 80:1; the weight space velocity of the fed olefin ranges from 0.1 h⁻¹ to 5 h⁻¹;
   preferably, the alkylation reaction conditions include: the reaction temperature ranges from 70 °C to 200 °C; the pressure ranges from 2 MPa to 4 MPa; the molar ratio of benzene/olefin ranges from 4:1 to 60:1; the weight space velocity of the fed olefin ranges from 0.3 h⁻¹ to 3 h⁻¹.
C14. The preparation method according to any one of Items C1-C13, wherein the oxygen-containing mixed gas is a mixture of an oxygen gas and a protective gas;
   preferably, the oxygen gas is present in an amount ranging from 1 vol.% to 50 vol.%, and preferably from 10 vol.% to 25 vol.% in the oxygen-containing mixed gas;
   preferably, the protective gas is selected from nitrogen gas, helium gas, argon gas, or combinations thereof;
   preferably, the regeneration temperature ranges from 400 °C to 700 °C, the regeneration time ranges from 4 h to 18 h, the volume space velocity of the oxygen-containing mixed gas ranges from 500 h⁻¹ to 6000 h⁻¹, the regeneration pressure ranges from 0.01 MPa to 0.1 MPa (gauge pressure).

### Examples

The present application will be described in detail below through examples.

In the following examples, unless otherwise specified, all raw materials used can be obtained from commercially.

In the following examples, the weight percentage of the bulk-modifying metal was measured by XRF, and the weight percentage of the surface-layer modifying metal was measured by XPS.

In the present application, an ESCALAB 250 X-ray photoelectron spectrometer (Thermo Fisher Scientific company) was used for the XPS test. The test conditions included: monochromatic Al Kα X-rays, energy 1486.6 eV, power 150 W, and charge shift correction using the C1s peak (284.8 eV) of contaminated carbon.

In the present application, a 3271 X-ray fluorescence spectrometer produced by Rigaku Corporation of Japan was used for the XRF test. The test conditions included: the anode target material of the X-ray tube was a rhodium target; the laser voltage was 50 kV; and the laser current was 50 mA.

In this application, a pyridine adsorption infrared test was performed using a Bruker Tensor II Fourier transform infrared spectrometer of the United States. The test procedure was performed as follows: a sample was made into a self-supporting wafer and placed in an in-situ cell of the infrared spectrometer and sealed, and the temperature was raised to 450°C at a rate of 10°C/min, and the system was vacuumized to about 10⁻⁶ Pa and maintained for 2 hrs to desorb impurities such as water molecules physically adsorbed by the molecular sieve. After cooling to room temperature, the static and saturation adsorptions of pyridine was conducted followed by balancing for 10 mins, then the temperature was raised to 200 °C, and the system was vacuumized to 10⁻⁶ Pa again, maintained for 30mins, and cooled to room temperature. The infrared spectrum was obtained by scanning in the range from 1000 cm⁻¹ to 4000 cm⁻¹.

The XRD test conditions included: the instrument used was an X-ray powder diffractometer from PANalytical of the Netherlands; the test conditions include: tube voltage 40 kV, tube current 40 mA, Cu target Kα radiation, scanning speed 2°/min, scanning range 2θ=5°-35°.

The molecular sieves used in the following inventive preparation examples of series I were purchased from Sinopec Catalyst Co., Ltd.

### Inventive Preparation Example I-1

1) 50 g of an HY molecular sieve (the silica/alumina molar ratio was 6, w(Na₂O)=0.0628%) was taken. The HY molecular sieve was subjected to a first ion exchange with a lanthanum nitrate solution. The concentration of lanthanum nitrate was 300 g/L. The exchange temperature was 80°C, the exchange time was 1 hour, and the weight ratio of the lanthanum nitrate solution to the HY molecular sieve was 3:1. After the exchange, the molecular sieve was filtered and dried, and then subjected to a first calcination at 550°C under a pressure of 0.02 MPa for 1 hour. During the calcination, 0.1 M ammonia solution was introduced at a rate of 0.1mL/min. A first exchanged molecular sieve was obtained after the calcination, and had a lanthanum metal weight percentage of 10.65% (obtained by measuring with XRF and calculation).
2) The first exchanged molecular sieve was subjected to a second ion exchange and a second calcination to produce a second exchanged molecular sieve, the conditions were identical to those in step 1. The second exchanged molecular sieve had a lanthanum metal weight percentage of 17.33% (obtained by measuring with XRF and calculation).
3) The second exchanged molecular sieve was calcined in a muffle furnace in an air atmosphere at 600 °C for 4 hrs to obtain a finished molecular sieve named Y1.

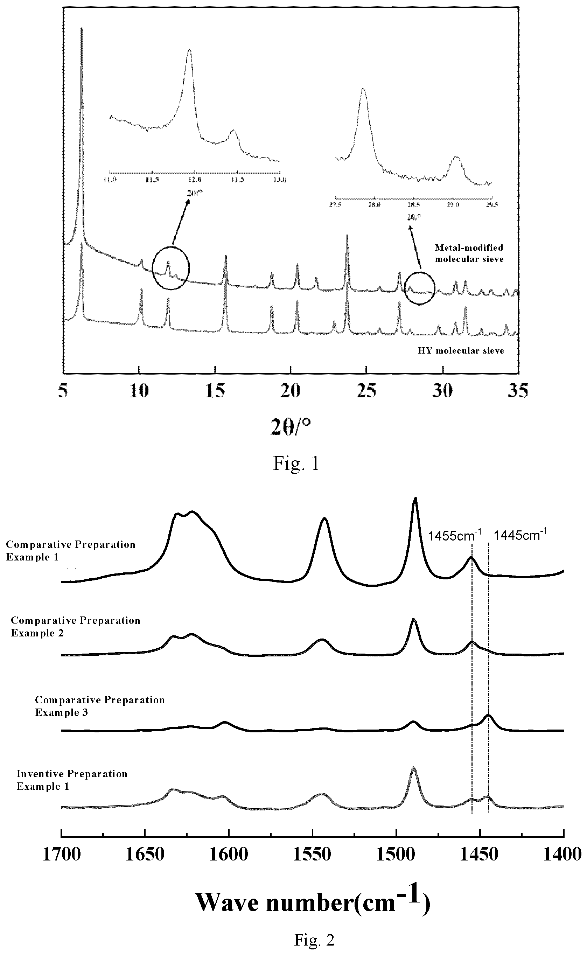

Through the XRD test, the XRD spectrum of the metal-modified molecular sieve Y1 is shown in Figure 1, wherein the characteristic peaks at 11.9° and 12.4° are attributed to the metal ions at the cation position, and the characteristic peak at 29.0° is attributed to the metal oxide on the surface of the molecular sieve; simultaneously, by comparing with the diffraction peaks of the HY molecular sieve, it can be seen that the peak positions of the corresponding characteristic peaks of the molecular sieve Y1 are identical to the peak positions of the diffraction peaks of the HY molecular sieve, that is, there is no shift, which proves that the modifying metal lanthanum is extra-framework of the molecular sieve.

Through the pyridine adsorption infrared test, the pyridine adsorption infrared spectrum of the metal-modified molecular sieve Y1 is shown in Figure 2, which shows remarkable absorption peaks at about 1455 cm⁻¹ and about 1445 cm⁻¹.

The measured physicochemical properties of the molecular sieve Y1 are shown in Table I-1.

### Inventive Preparation Example I-2

The procedure in Inventive Preparation Example I-1 was followed, except that the HY molecular sieve in step 1) was replaced with an equal weight of an Hβ molecular sieve (the silica/alumina molar ratio was 19, w (Na₂O)=0.0171%). The obtained metal-modified molecular sieve was named Y2.

The measured physicochemical properties of the molecular sieve Y2 are shown in Table I-1.

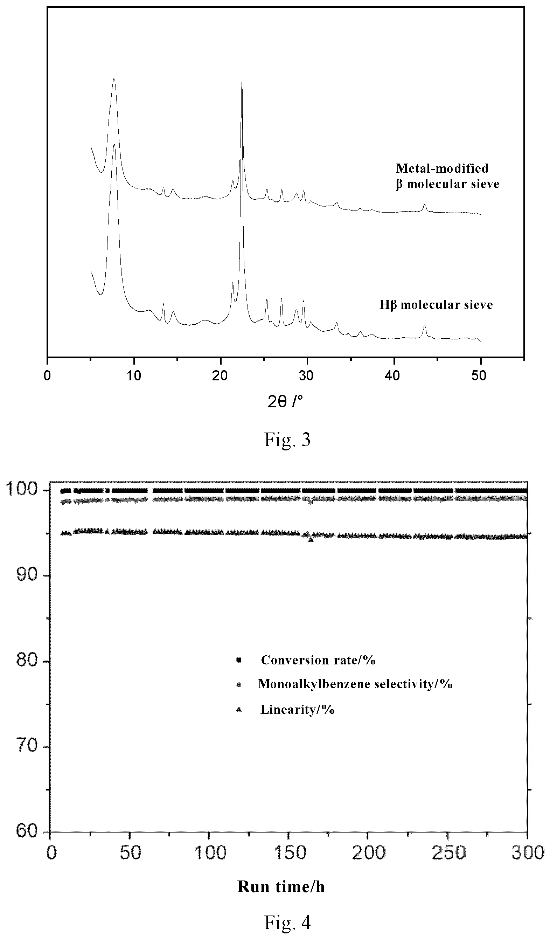

Through the XRD test, the XRD spectrum of the metal-modified molecular sieve Y2 is shown in Figure 3; by comparing with the diffraction peaks of the Hβ molecular sieve, it can be seen that the peak positions of the corresponding characteristic peaks of the molecular sieve Y2 are identical to the peak positions of the diffraction peaks of the Hβ molecular sieve, that is, there is no shift, which proves that the modifying metal lanthanum is extra-framework of the molecular sieve.

### Inventive Preparation Example I-3

The procedure in Inventive Preparation Example I-1 was followed, except that the lanthanum nitrate solution was replaced with a cerium nitrate solution. The obtained metal-modified molecular sieve was named Y3.

The measured physicochemical properties of the molecular sieve Y3 are shown in Table I-1.

### Inventive Preparation Example I-4

The procedure in Inventive Preparation Example I-1 was followed, except that the lanthanum nitrate solution was replaced with a strontium nitrate solution. The obtained metal-modified molecular sieve was named Y4.

The measured physicochemical properties of the molecular sieve Y4 are shown in Table I-1.

### Inventive Preparation Example I-5

The procedure in Inventive Preparation Example I-1 was followed, except that in the first and second ion exchanges, the weight ratio of the lanthanum nitrate solution to the HY molecular sieve was 2.5:1. The obtained metal-modified molecular sieve was named Y5.

The measured physicochemical properties of the molecular sieve Y5 are shown in Table I-1.

### Inventive Preparation Example I-6

The procedure in Inventive Preparation Example I-1 was followed, except that in the first and second ion exchanges, the weight ratio of the lanthanum nitrate solution to the HY molecular sieve was 3.8:1. The obtained metal-modified molecular sieve was named Y6.

The measured physicochemical properties of the molecular sieve Y6 are shown in Table I-1.

### Inventive Preparation Example I-7

The procedure in Inventive Preparation Example I-1 was followed, except that the introduction rate of ammonia solution was replaced with 0.03 mL/min. The obtained metal-modified molecular sieve was named Y7.

The measured physicochemical properties of the molecular sieve Y7 are shown in Table I-1.

### Inventive Preparation Example I-8

1) 50 g of a NaY molecular sieve (the silica/alumina molar ratio was 5, w(Na₂O)=12.8%) was taken. The NaY molecular sieve was subjected to a first ion exchange with a mixed solution of ammonium chloride and lanthanum nitrate. The concentration of ammonium chloride was 120 g/L (calculated separately). The concentration of lanthanum nitrate was 300 g/L (calculated separately). The exchange temperature was 80 °C, the exchange time was 1 hour, and the weight ratio of the mixed solution to the molecular sieve was 3:1. After the exchange, the molecular sieve was filtered and dried, and then subjected to a first calcination at 550°C under a pressure of 0.02 MPa for 1 hour. During the calcination, 0.1M ammonia solution was introduced at a rate of 0.1 mL/min. A first exchanged molecular sieve was obtained after the calcination.
2) The first exchanged molecular sieve was subjected to a second ion exchange and a second calcination to produce a second exchanged molecular sieve, the conditions were identical to those in step 1.
3) The second exchanged molecular sieve was exchanged with an ammonium chloride solution. The concentration of ammonium chloride was 120 g/L. The exchange temperature was 80 °C, the exchange time was 1 hour, and the weight ratio of the ammonium chloride solution to the molecular sieve was 3:1. After the exchange, the molecular sieve was filtered and dried, and then exchanged with an ammonium chloride solution again under the same conditions as above. This process was repeated twice, so that the weight percentage of Na in the molecular sieve was less than 0.2%. The second exchanged molecular sieve was calcined in a muffle furnace in an air atmosphere at 600°C for 4 hours, to obtain a finished molecular sieve named Y8. The measured physicochemical properties of the molecular sieve Y8 are shown in Table I-1.

### Inventive Preparation Example I-9

The procedure in Inventive Preparation Example I-1 was followed, except that the lanthanum nitrate solution had a concentration of 400 g/L. The obtained metal-modified molecular sieve was named Y9.

The measured physicochemical properties of the molecular sieve Y9 are shown in continued Table I-1.

### Inventive Preparation Example I-10

The procedure in Inventive Preparation Example I-1 was followed, except that the lanthanum nitrate solution had a concentration of 200 g/L. The obtained metal-modified molecular sieve was named Y10.

The measured physicochemical properties of the molecular sieve Y10 are shown in continued Table I-1.

### Inventive Preparation Example I-11

The procedure in Inventive Preparation Example I-1 was followed, except that the HY molecular sieve in step 1) was replaced with an equal weight of an HX molecular sieve (the silica/alumina molar ratio was 2, w(Na₂O)=0.0156%). The obtained metal-modified molecular sieve was named Y11.

The measured physicochemical properties of the molecular sieve Y11 are shown in continued Table I-1.

### Inventive Preparation Example I-12

1) 50 g of an HY molecular sieve (the silica/alumina molar ratio was 6, w(Na₂O)=0.0628%) was taken. The HY molecular sieve was subjected to a first ion exchange with a lanthanum nitrate solution. The concentration of lanthanum nitrate was 300 g/L. The exchange temperature was 80 °C, the exchange time was 1 hour, and the weight ratio of the lanthanum nitrate solution to the HY molecular sieve was 3:1. After the exchange, the molecular sieve was filtered and dried, and then subjected to a first calcination at 550°C under a pressure of 0.02 MPa for 1 hour. During the calcination, 0.1M ammonia solution was introduced at a rate of 0.1mL/min to obtain a first exchanged molecular sieve.
2) The first exchanged molecular sieve was subjected to a second ion exchange. Being different from the first ion exchange, the temperature of the second ion exchange was 90°C. After the calcination, a second exchanged molecular sieve was obtained.
3) The second exchanged molecular sieve was calcined in a muffle furnace in an air atmosphere at 600°C for 4 hrs to obtain a finished molecular sieve named Y12.

The measured physicochemical properties of the molecular sieve Y12 are shown in continued Table I-1.

### Comparative Preparation Example I-1

An untreated HY molecular sieve in Inventive Preparation Example I-1 was used and named DY1. The measured physicochemical properties of the molecular sieve DY1 are shown in continued Table I-1.

Through the pyridine adsorption infrared test, the pyridine adsorption infrared spectrum of the molecular sieve DY1 is shown in Figure 2, in which only one absorption peak is shown in the range from 1440 cm⁻¹ to 1460 cm⁻¹.

### Comparative Preparation Example I-2

1) 50 g of an HY molecular sieve (the silica/alumina molar ratio was 6, w(Na₂O)=0.0628%) was taken. The HY molecular sieve was subjected to a first ion exchange with a lanthanum nitrate solution. The concentration of lanthanum nitrate was 150 g/L. The exchange temperature was 80 °C, the exchange time was 1 hour, and the weight ratio of the solution to the molecular sieve was 3:1. After the exchange, the molecular sieve was filtered and dried, and then subjected to a calcination at 550 °C under a pressure of 0.02 MPa for 1 hour. Meanwhile, during the calcination, 0.1M ammonia solution was introduced at a rate of 0.1mL/min. A first exchanged molecular sieve was obtained after the calcination, and had a lanthanum metal weight percentage of 5.83% (obtained by measuring with XRF and calculation).
2) The first exchanged molecular sieve was subjected to a second ion exchange and a second calcination to produce a second exchanged molecular sieve, the conditions were identical to those in step 1. The second exchanged molecular sieve had a lanthanum metal weight percentage of 6.97% (obtained by measuring with XRF and calculation).
3) The second exchanged molecular sieve was calcined in a muffle furnace in an air atmosphere at 600 °C for 4 hrs to obtain a finished molecular sieve named DY2.

Through the pyridine adsorption infrared test, the pyridine adsorption infrared spectrum of the molecular sieve DY2 is shown in Figure 2, in which a remarkable absorption peak is shown only at about 1455 cm⁻¹.

The measured physicochemical properties of the molecular sieve DY2 are shown in continued Table I-1.

### Comparative Preparation Example I-3

1) 50 g of an HY molecular sieve (the silica/alumina molar ratio was 6, w(Na₂O)=0.0628%) was taken. The HY molecular sieve was subjected to a first ion exchange with a lanthanum nitrate solution. The concentration of lanthanum nitrate was 900 g/L. The exchange temperature was 80 °C, the exchange time was 1 hour, and the weight ratio of the solution to the molecular sieve was 3:1. After the exchange, the molecular sieve was evaporated to remove water and dried, and then subjected to a calcination at 550°C under a pressure of 0.02 MPa for 1 hour. Meanwhile, during the calcination, 0.1M ammonia solution was introduced at a rate of 0.1mL/min. The calcined molecular sieve had a lanthanum metal weight percentage of 19.06% (obtained by measuring with XRF and calculation).
2) The first exchanged molecular sieve was subjected to a second ion exchange and a second calcination to produce a second exchanged molecular sieve, the process was identical to that in step 1. The second exchanged molecular sieve had a lanthanum metal weight percentage of 31.80% (obtained by measuring with XRF and calculation).
3) The second exchanged molecular sieve was calcined in a muffle furnace in an air atmosphere at 600 °C for 4 hrs to obtain a finished molecular sieve named DY3.

Through the pyridine adsorption infrared test, the pyridine adsorption infrared spectrum of the molecular sieve DY3 is shown in Figure 2, in which a remarkable absorption peak is shown only at about 1445 cm⁻¹.

The measured physicochemical properties of the molecular sieve DY3 are shown in continued Table I-1.

### Comparative Preparation Example I-4

The procedure in Preparation Example I-1 was followed, except that the same amount of water was introduced to replace the ammonia solution during the first and second calcinations. The obtained molecular sieve was named as DY4. The measured physicochemical properties of the molecular sieve DY4 are shown in continued Table I-1.

### Comparative Preparation Example I-5

This comparative example illustrates a process of preparing a molecular sieve by loading a metal by vacuum impregnation. 50 g of an HY molecular sieve (the silica/alumina molar ratio was 6, w(Na₂O)=0.0628%) was taken. The HY molecular sieve was subjected to an excessive vacuum impregnation with a lanthanum nitrate solution. The loading amount of lanthanum was 20%, and the weight ratio of the lanthanum nitrate solution to the HY molecular sieve was 2:1. A rotary impregnation was conducted with a rotary evaporator at 80 °C for 3 hours, and then water was removed in vacuum. The impregnated catalyst was dried at 120 °C for 12 hours. The dried catalyst was calcined at 600 °C in an air atmosphere in a muffle furnace for 4 hours. The obtained molecular sieve was named DY5. The measured physicochemical properties of the molecular sieve DY5 are shown in continued Table I-1.

**Table I-1: Properties of the catalysts obtained in Inventive Preparation Examples I-1 to I-12 and Comparative Preparation Examples I-1 to I-5**

| Example No. | Inventive Preparation Example I-1 | Inventive Preparation Example I-2 | Inventive Preparation Example I-3 | Inventive Preparation Example I-4 | Inventive Preparation Example I-5 | Inventive Preparation Example I-6 | Inventive Preparation Example I-7 | Inventive Preparation Example I-8 |
|---|---|---|---|---|---|---|---|---|
| Sample No. | Y1 | Y2 | Y3 | Y4 | Y5 | Y6 | Y7 | Y8 |
| Silica/alumina molar ratio | 6.20 | 19.20 | 6.18 | 6.31 | 6.21 | 6.24 | 6.27 | 5.28 |
| Metal | La | La | Ce | Sr | La | La | La | La |
| Metal atomic radius/pm | 187.91 | 187.91 | 183.21 | 215.1 | 187.91 | 187.91 | 187.91 | 187.91 |
| Metal content/wt.% | 17.33 | 17.21 | 17.48 | 16.85 | 13.26 | 18.94 | 11.89 | 17.51 |
| Weight percentage of bulk modifying metal/% | 17.33 | 17.21 | 17.48 | 16.85 | 13.26 | 18.94 | 11.89 | 17.51 |
| Weight percentage of the surface-layer modifying metal/% | 22.07 | 20.31 | 21.68 | 21.06 | 12.20 | 25.38 | 13.44 | 17.69 |
| Weight percentage of the surface-layer modifying metal / weight percentage of the bulk modifying metal | 1.27 | 1.18 | 1.24 | 1.25 | 0.92 | 1.34 | 1.13 | 1.01 |
| Strong L acid ratio* | 0.53 | 0.51 | 0.52 | 0.52 | 0.45 | 0.55 | 0.50 | 0.47 |

| | | | | | | | | |
|---|---|---|---|---|---|---|---|---|
| * Strong L acid ratio means a ratio of the area of the absorption peak at about 1445 cm⁻¹ to the total area of the absorption peaks at both about 1445 cm⁻¹ and about 1450 cm⁻¹ in the pyridine adsorption infrared spectrum. | | | | | | | | |

**Continued Table 1-1: Properties of the catalysts obtained in Inventive Preparation Examples I-1 to I-12 and Comparative Preparation Examples I-1 to I-5**

| Example No. | Inventive Preparation Example I-9 | Inventive Preparation Example I-10 | Inventive Preparation Example I-11 | Inventive Preparation Example I-12 | Comparative Preparation Example I-1 | Comparative Preparation Example I-2 | Comparative Preparation Example I-3 | Comparative Preparation Example I-4 | Comparative Preparation Example I-5 |
|---|---|---|---|---|---|---|---|---|---|
| Sample No. | Y9 | Y10 | Y11 | Y12 | DY1 | DY2 | DY3 | DY4 | DY5 |
| Silica/alumina molar ratio | 6.26 | 6.24 | 2.06 | 6.19 | 6 | 6.22 | 6.20 | 6.26 | 6.28 |
| Metal | La | La | La | La | / | La | La | La | La |
| Metal atomic radius/pm | 187.91 | 187.91 | 187.91 | 187.91 | / | 187.91 | 187.91 | 187.91 | 187.91 |
| Metal content/wt.% | 22.93 | 12.12 | 17.62 | 18.51 | / | 6.97 | 31.80 | 9.78 | 20.12 |
| Weight percentage of bulk modifying metal/% | 22.93 | 12.12 | 17.62 | 18.51 | / | 6.97 | 31.80 | 9.78 | 20.12 |
| Weight percentage of the surface-layer modifying metal/% | 28.66 | 11.88 | 22.21 | 22.59 | / | 6.21 | 47.43 | 16.14 | 35.92 |
| Weight percentage of the surface-layer modifying metal / weight percentage of the bulk modifying metal | 1.25 | 0.98 | 1.26 | 1.22 | / | 0.89 | 1.49 | 1.65 | 1.79 |
| Strong L acid ratio* | 0.52 | 0.46 | 0.52 | 0.51 | 0 | 0.12 | 0.81 | 0.32 | 0.28 |

### Inventive Application Example I-1

An alkylation reaction of benzene with 1-dodecene, using the molecular sieve sample Y1 of Inventive Preparation Example I-1, was performed under the reaction conditions of 120 °C and 2.5 MPa; the olefin feed space velocity was 0.354 h⁻¹, and the benzene/olefin molar ratio was 60. The conversion rate and the product selectivity under this reaction condition are shown in Table I-2.

The molecular sieve of Inventive Preparation Example I-1 was evaluated for the stability, and the results are shown in Figure 4. As can be seen from Figure 4, the catalytic performance of the molecular sieve prepared in Inventive Preparation Example I-1 was stable in the alkylation reaction of 1-dodecene and benzene. In the 300 h evaluation, the selectivity of monoalkylbenzene was close to 100% all the while, and the alkylbenzene linearity was 94% or higher all the while too. 1-dodecene conversion rate (%) = (the molar amount of 1-dodecene before the reaction - the molar amount of 1-dodecene after the reaction)/the molar amount of 1-dodecene before the reaction × 100%; Monoalkylbenzene selectivity (%) = the molar amount of monoalkylbenzene after the reaction/the molar amount of all reaction products × 100%; Linearity (%) = the molar amount of the linear alkylbenzene after the reaction /the molar amount of monoalkylbenzene after the reaction × 100%.

### Inventive Application Examples I-2 to I-12

The procedure in Inventive Application Example I-1 was followed, except that the molecular sieves of Inventive Preparation Examples I-2 to I-12 were respectively used to replace Y1. The alkylation reaction results are shown in Table I-2 and Continued Table I-2.

### Comparative Application Examples I-1 to I-5

The procedure in Inventive Application Example I-1 was followed, except that the molecular sieves of Comparative Preparation Examples I-1 to I-5 were respectively used to replace Y1. The alkylation reaction results are shown in Continued Table I-2.

**Table I-2: Reaction results of Inventive Application Examples I-1 to I-12 and Comparative Application Examples I-1 to I-5**

| Test Example No. | Inventive Application Example I-1 | Inventive Application Example I-2 | Inventive Application Example I-3 | Inventive Application Example I-4 | Inventive Application Example I-5 | Inventive Application Example I-6 | Inventive Application Example I-7 | Inventive Application Example I-8 |
|---|---|---|---|---|---|---|---|---|
| Molecular Sieve No. | Y1 | Y2 | Y3 | Y4 | Y5 | Y6 | Y7 | Y8 |

| 20 h | | | | | | | | |
|---|---|---|---|---|---|---|---|---|
| 1-dodecene conversion rate/% | 100 | 99.3 | 99.7 | 99.9 | 100 | 100 | 99.7 | 99.9 |
| Monoalkylbenzene selectivity/% | 99.0 | 99.1 | 99.0 | 99.2 | 99.1 | 99.0 | 99.0 | 99.1 |
| Linearity/% | 94.5 | 94.0 | 94.4 | 94.2 | 94.4 | 94.6 | 94.1 | 94.3 |

| 100 h | | | | | | | | |
|---|---|---|---|---|---|---|---|---|
| 1-dodecene conversion rate/% | 100 | 82.3 (50 h) | 100 | 100 | 100 | 100 | 99.8 | 100 |
| Monoalkylbenzene selectivity/% | 99.2 | 97.8 (50 h) | 99.3 | 99.1 | 99.2 | 99.1 | 99.1 | 99.2 |
| Linearity/% | 94.5 | 95.2 (50 h) | 94.5 | 94.2 | 94.3 | 94.6 | 94.1 | 94.4 |

| 150 h | | | | | | | | |
|---|---|---|---|---|---|---|---|---|
| 1-dodecene conversion rate/% | 100 | / | 100 | 100 | 100 | 100 | 99.8 | 100 |
| Monoalkylbenzene selectivity/% | 99.2 | / | 99.2 | 99.1 | 99.2 | 99.1 | 99.2 | 99.2 |
| Linearity/% | 94.3 | / | 94.4 | 94.2 | 94.3 | 94.5 | 94.0 | 94.3 |

**Continued Table I-2: Reaction results of Inventive Application Examples I-1 to I-12 and Comparative Application Examples I-1 to I-5**

| Test Example No. | Inventive Application Example I-9 | Inventive Application Example I-10 | Inventive Application Example I-11 | Inventive Application Example I-12 | Comparative Application Example I-1 | Comparative Application Example I-2 | Comparative Application Example I-3 | Comparative Application Example I-4 | Comparative Application Example I-5 |
|---|---|---|---|---|---|---|---|---|---|
| Molecular Sieve No. | Y9 | Y10 | Y11 | Y12 | DY1 | DY2 | DY3 | DY4 | DY5 |

| 20 h | | | | | | | | | |
|---|---|---|---|---|---|---|---|---|---|
| 1-dodecene conversion rate/% | 100 | 100 | 100 | 100 | 100 | 99.9 | 86.3 (10 h) | 99.8 | 88.5 |
| Monoalkylbenzene selectivity/% | 99.4 | 99.1 | 99.2 | 99.3 | 99.5 | 96.0 | 84.3 (10 h) | 99.1 | 89.3 |
| Linearity/% | 94.7 | 94.4 | 94.4 | 94.6 | 92.8 | 92.1 | 96.1 (10 h) | 93.4 | 92.5 |

| 100 h | | | | | | | | | |
|---|---|---|---|---|---|---|---|---|---|
| 1-dodecene conversion rate/% | 100 | 100 | 100 | 100 | 92.4 | / | / | 94.4 | |
| Monoalkylbenzene selectivity/% | 99.5 | 99.2 | 99.3 | 99.4 | 91.6 | / | / | 93.2 | |
| Linearity/% | 94.7 | 94.4 | 94.4 | 94.6 | 95.0 | / | / | 94.3 | |

| 150 h | | | | | | | | | |
|---|---|---|---|---|---|---|---|---|---|
| 1-dodecene conversion rate/% | 100 | 100 | 100 | 100 | / | / | / | 72.7 | |
| Monoalkylbenzene selectivity/% | 99.5 | 99.2 | 99.2 | 99.4 | / | / | / | 56.2 | |
| Linearity/% | 94.6 | 94.4 | 94.4 | 94.6 | / | / | / | 95.8 | |

It can be seen from the results in Table I-2 and continued Table I-2 that the metal-modified molecular sieves prepared in the inventive preparation examples of series I of the present application had high activity, stability and selectivity in catalyzing the alkylation reaction of 1-dodecene and benzene, wherein the selectivity of monoalkylbenzene was 98% or higher and the alkylbenzene linearity arrived at 94% or higher.

By comparing Inventive Preparation Example I-1 with Comparative Preparation Examples I-1 to I-3, it can be seen that when no metal modification was performed, the metal content was too high or too low, it was not conducive to improving the catalyst life and the product linearity, especially when the metal content exceeded 30 wt.%, the olefin conversion rate was greatly reduced, and the monoalkylbenzene selectivity was poor. By comparing Inventive Preparation Example I-1 with Comparative Preparation Example I-4, it can be seen that when the first calcination and the second calcination were not performed using an alkaline atmosphere, the weight percentage of the surface-layer modifying metal was in a higher proportion, the catalyst activity was reduced, and the alkylbenzene linearity was reduced. Simultaneously, as the reaction proceeded, the catalytic effect rapidly diminished, resulting in a short catalyst lifetime.

The following inventive preparation examples of series II are used to illustrate the preparation of the solid acid catalysts of the present application.

### Inventive Preparation Example II-1

The molecular sieve sample Y1 and pseudo-boehmite (with an average particle size of 100 µm) were taken and mixed in a weight percentage of 80:20 on a dry basis; 3 wt.% of sesbania powder and 3 wt.% of nitric acid were added respectively (based on the total weight on a dry basis of the molecular sieve and pseudo-boehmite), and deionized water was added in a weight ratio of 1:1 based on a dry basis of the molecular sieve and pseudo-boehmite. These materials were mixed well and shaped through extrusion, dried at 110 °C for 6 h so that the dry basis weight of the resulting mixed shaped bodies after drying was 65 wt.%, and then the shaped bodies were calcined in air at 550 °C for 4 h to produce a solid acid catalyst G1.

### Inventive Preparation Examples II-2 to II-12

The procedure in Inventive Preparation Example II-1 was followed, except that the metal-modified molecular sieves of Inventive Preparation Examples I-2 to I-12 were respectively used to replace Y1 to produce solid acid catalysts G2 to G12.

### Inventive Preparation Example II-13

The catalyst preparation procedure in Inventive Preparation Example II-1 was replaced as follows: the molecular sieve sample Y1 and silica (with an average particle size of 0.05 µm) were taken and mixed in a weight percentage of 80:20 on a dry basis; 3 wt.% of sesbania powder and 3 wt.% of nitric acid were added respectively (based on the total weight on a dry basis of the molecular sieve and silica), and deionized water was added in a weight ratio of 0.5:1 based on the dry basis of the molecular sieve and silica. These materials were mixed well and shaped through extrusion, dried at 150 °C for 6 h so that the dry basis weight of the resulting mixed shaped bodies after drying was 70 wt.%, and then the shaped bodies were calcined in air at 600 °C for 6 h to produce a solid acid catalyst G13.

### Inventive Preparation Example II-14

The procedure in Inventive Preparation Example II-1 was followed, except that the molecular sieve sample Y1 and pseudo-boehmite were mixed in a weight percentage of 60:40 on a dry basis to produce a solid acid catalyst G14.

### Comparative Preparation Example II-1 to II-4

The procedure in Inventive Preparation Example II-1 was followed, except that the molecular sieves DY-1 to DY-4 of Comparative Preparation Examples I-1 to I-4 were respectively used to replace Y1 to produce solid acid catalysts DG1 to DG4.

### Inventive Application Example II-1

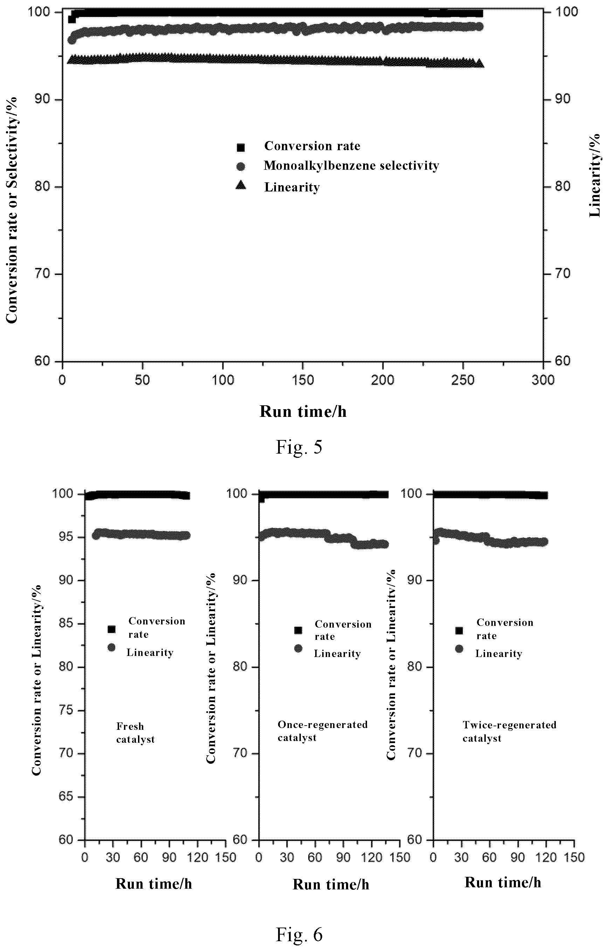

An alkylation reaction of benzene with 1-dodecene, using the solid acid catalyst G1 obtained in Inventive Preparation Example II-1, was performed under the reaction conditions of 120 °C and 3 MPa; the olefin feed space velocity was 0.354 h⁻¹, and the fed benzene/olefin molar ratio was 60:1. The results such as the 1-dodecene conversion rate and the monoalkylbenzene selectivity obtained through calculation are shown in Table II-1. The stable operation evaluation results are shown in Figure 5. It can be seen from Figure 5 that in the 260 h stable operation evaluation, the monoalkylbenzene selectivity was close to 100% all the while, and the alkylbenzene linearity was 94% or higher all the while too.

When the 1-dodecene conversion rate was lower than 98%, the catalyst was regenerated at 500 °C for 8 h with an oxygen-containing mixed gas (having an oxygen gas content of 20% and a nitrogen gas content of 80%), the regeneration pressure was 0.01 MPa (gauge pressure), and the volume space velocity of the mixed gas was 3000 h⁻¹; this was recorded as one reaction-regeneration procedure, and after the regeneration was completed, the reaction was carried out again under the same reaction conditions, and a total of two reaction-regeneration procedures were carried out. The reaction results for the fresh catalyst, the once-regenerated catalyst and the twice-regenerated catalyst are shown in Table II-2.

The continuous reaction-regeneration operation evaluation results are shown in Figure 6. It can be seen from Figure 6 that in two reaction-regeneration evaluations, the monoalkylbenzene selectivity was close to 100% all the while, and the alkylbenzene linearity was 94% or higher all the while too. Among them, 1-dodecene conversion rate (%) = (the molar amount of 1-dodecene before the reaction - the molar amount of 1-dodecene after the reaction)/the molar amount of 1-dodecene before the reaction × 100%; Monoalkylbenzene Selectivity (%) = the molar amount of monoalkylbenzene after the reaction/the molar amount of all reaction products × 100%; Linearity (%) = the molar amount of the linear alkylbenzene after the reaction /the molar amount of monoalkylbenzene after the reaction × 100%.

### Inventive Application Examples II-2 to II-14

The procedure in Inventive Application Example II-1 was followed, except that the solid acid catalysts G2 to G14 of Inventive Preparation Examples II-2 to II-14 were respectively used to replace G1 and perform the alkylation reaction. The results are shown in Table II-1.

### Inventive Application Example II-15

The method described in Inventive Application Example II-1 was followed, except that the regeneration temperature was replaced with 400 °C. A total of two reaction-regeneration procedures were conducted. The reaction results for the fresh catalyst, once-regenerated catalyst, and twice-regenerated catalyst are listed in continued Table II-2.

### Inventive Application Example II-16

The method described in Inventive Application Example II-1 was followed, except that the alkylation reaction temperature was replaced with 220 °C. A total of two reaction-regeneration procedures were conducted. The reaction results for the fresh catalyst, once-regenerated catalyst, and twice-regenerated catalyst are listed in continued Table II-2.

### Comparative Application Example II-1

The procedure in Inventive Application Example II-1 was followed, except that the solid acid catalysts DG1 of Comparative Preparation Example II-1 was used to replace G1 and perform the alkylation reaction. The result is shown in Table II-1.

When the 1-dodecene conversion rate was lower than 98%, the solid acid catalyst was regenerated by the regeneration method in Inventive Application Example II-1, and a total of two reaction-regeneration procedures were conducted. The reaction results for the fresh catalyst, once-regenerated catalyst, and twice-regenerated catalyst are listed in continued Table II-2.

### Comparative Application Examples II-2 to II-4

The procedure in Inventive Application Example II-1 was followed, except that the solid acid catalysts DG2 to DG4 of Comparative Preparation Examples II-2 to II-4 were respectively used to replace G1 and perform the alkylation reaction. The results are shown in Table II-1.

**Table II-1: Reaction results of Inventive Application Examples II-1 to II-14 and Comparative Application Examples II-1 to II-4**

| | Inventive Application Example II-1 | Inventive Application Example II-2 | Inventive Application Example II-3 | Inventive Application Example II-4 | Inventive Application Example II-5 | Inventive Application Example II-6 | Inventive Application Example II-7 | Inventive Application Example II-8 | Inventive Application Example II-9 |
|---|---|---|---|---|---|---|---|---|---|
| 40 h | | | | | | | | | |
| 1-dodecene conversion rate/% | 100 | 99.15 (20 h) | 99.97 | 100 | 100 | 100 | 99.75 | 99.94 | 100 |
| Monoalkylbenzene selectivity/% | 99.14 | 99.17 (20 h) | 99.12 | 99.25 | 99.12 | 99.16 | 99.10 | 99.12 | 99.52 |
| Linearity/% | 95.30 | 94.61 (20 h) | 95.19 | 95.15 | 95.21 | 95.41 | 94.72 | 95.03 | 95.48 |

| 80 h | | | | | | | | | |
|---|---|---|---|---|---|---|---|---|---|
| 1-dodecene conversion rate/% | 100 | 83.63 (40 h) | 100 | 100 | 100 | 100 | 99.91 | 100 | 100 |
| Monoalkylbenzene selectivity/% | 99.32 | 98.03 (40 h) | 99.30 | 99.35 | 99.34 | 99.28 | 99.26 | 99.30 | 99.61 |
| Linearity/% | 95.21 | 95.28 (40 h) | 95.20 | 95.17 | 95.26 | 95.43 | 94.79 | 95.14 | 95.52 |

| 108 h | | | | | | | | | |
|---|---|---|---|---|---|---|---|---|---|
| 1-dodecene conversion rate/% | 99.85 | - | 99.84 | 100 | 100 | 100 | 99.90 | 100 | 100 |
| Monoalkylbenzene selectivity/% | 99.28 | - | 99.21 | 99.27 | 99.29 | 99.21 | 99.24 | 99.39 | 99.62 |
| Linearity/% | 95.21 | - | 95.19 | 95.15 | 95.24 | 95.41 | 94.78 | 95.17 | 95.49 |

**Continued Table II-1: Reaction results of Inventive Application Examples II-1 to II-14 and Comparative Application Examples II-1 to II-4**

| | Inventive Application Example II-10 | Inventive Application Example II-11 | Inventive Application Example II-12 | Inventive Application Example II-13 | Inventive Application Example II-14 | Comparative Application Example II-1 | Comparative Application Example II-2 | Comparative Application Example II-3 | Comparative Application Example II-4 |
|---|---|---|---|---|---|---|---|---|---|
| 40 h | | | | | | | | | |
| 1-dodecene conversion rate/% | 100 | 100 | 99.97 | 99.86 | 99.54 | 99.98 | 99.89 | 79.88 (15 h) | 99.87 |
| Monoalkylbenzene selectivity/% | 99.22 | 99.18 | 99.24 | 99.27 | 99.01 | 98.59 | 95.48 | 78.54 (15 h) | 99.23 |
| Linearity/% | 95.31 | 95.32 | 95.35 | 95.10 | 93.95 | 91.17 | 90.82 | 96.83 (15 h) | 93.89 |

| 80 h | | | | | | | | | |
|---|---|---|---|---|---|---|---|---|---|
| 1-dodecene conversion rate/% | 100 | 100 | 100 | 100 | 99.74 | 99.97 | 96.87 | - | 93.56 |
| Monoalkylbenzene selectivity/% | 99.38 | 99.33 | 99.36 | 99.32 | 99.25 | 98.80 | 92.42 | - | 90.30 |
| Linearity/% | 95.29 | 95.31 | 95.37 | 95.19 | 94.38 | 90.67 | 91.51 | - | 94.83 |

| 108 h | | | | | | | | | |
|---|---|---|---|---|---|---|---|---|---|
| 1-dodecene conversion rate/% | 100 | 100 | 100 | 100 | 88.92 | 85.25 | 73.12 | - | 70.12 |
| Monoalkylbenzene selectivity/% | 99.36 | 99.30 | 99.37 | 99.29 | 86.51 | 89.79 | 63.55 | - | 62.41 |
| Linearity/% | 95.29 | 95.32 | 95.34 | 95.15 | 95.30 | 91.63 | 92.98 | - | 95.51 |

It can be seen from the results in Table II-1 and continued Table II-1 that the solid acid catalysts prepared in the inventive preparation examples of series II of the present application had relatively high catalytic activity. In the alkylation reaction of 1-dodecene and benzene, the conversion rate of the reactants was high, and the monoalkylbenzene selectivity was 98% or higher, and the alkylbenzene linearity arrived at 94% or higher. In the 260 h stable operation evaluation, the monoalkylbenzene selectivity and the alkylbenzene linearity were stably good, which can be suitable for large-scale industrial applications.

**Table II-2: Operation Results for continuous reaction-regeneration of Inventive Application Example II-1 and Comparative Application Example II-1**

| | Inventive Application Example II-1 fresh catalyst | Inventive Application Example II-1 once-regenerated catalyst | Inventive Application Example II-1 twice-regenerated catalyst | Comparative Application Example II-1 fresh catalyst | Comparative Application Example II-1 once-regenerated catalyst | Comparative Application Example II-1 twice-regenerated catalyst |
|---|---|---|---|---|---|---|
| 40 h | | | | | | |
| 1-dodecene conversion rate/% | 100 | 99.99 | 99.99 | 99.98 | 99.97 | 99.87 |
| Monoalkylbenzene selectivity/% | 99.14 | 98.75 | 98.59 | 98.59 | 97.47 | 97.64 |
| Linearity/% | 95.30 | 95.46 | 95.08 | 91.17 | 90.03 | 90.92 |

| 80 h | | | | | | 78h |
|---|---|---|---|---|---|---|
| 1-dodecene conversion rate/% | 100 | 99.98 | 99.93 | 99.97 | 99.58 | 98.69 |
| Monoalkylbenzene selectivity/% | 99.32 | 98.86 | 98.93 | 98.80 | 96.37 | 87.28 |
| Linearity/% | 95.21 | 94.83 | 94.30 | 90.67 | 91.46 | 92.08 |

| 108 h | | | | | | |
|---|---|---|---|---|---|---|
| 1-dodecene conversion rate/% | 99.85 | 99.99 | 99.91 | 85.25 | no activity | no activity |
| Monoalkylbenzene selectivity/% | 99.28 | 99.11 | 98.97 | 89.79 | | |
| Linearity/% | 95.21 | 94.13 | 94.46 | 91.63 | | |

**Continued Table II-2: Operation Results for continuous reaction-regeneration of Inventive Application Examples II-15 and II-16**

| | Inventive Application Example II-15 fresh catalyst | Inventive Application Example II-15 once-regenerated catalyst | Inventive Application Example II-15 twice-regenerated catalyst | Inventive Application Example II-16 fresh catalyst | Inventive Application Example II-16 once-regenerated catalyst | Inventive Application Example II-16 twice-regenerated catalyst |
|---|---|---|---|---|---|---|
| 40 h | | | | | | |
| 1-dodecene conversion rate/% | 100 | 98.85 | 98.89 | 99.84 | 99.86 | 99.82 |
| Monoalkylbenzene selectivity/% | 99.14 | 99.26 | 99.35 | 98.98 | 98.56 | 98.41 |
| Linearity/% | 95.30 | 93.89 | 94.10 | 93.88 | 93.52 | 93.67 |

| 80 h | | | | | | |
|---|---|---|---|---|---|---|
| 1-dodecene conversion rate/% | 100 | 98.95 | 98.99 | 99.89 | 99.82 | 99.85 |
| Monoalkylbenzene selectivity/% | 99.32 | 99.40 | 99.49 | 99.02 | 99.00 | 99.01 |
| Linearity/% | 95.21 | 94.02 | 94.21 | 94.05 | 93.63 | 93.91 |

| 108 h | | | | | | |
|---|---|---|---|---|---|---|
| 1-dodecene conversion rate/% | 99.85 | 98.99 | 98.86 | 99.90 | 99.92 | 99.89 |
| Monoalkylbenzene selectivity/% | 99.28 | 99.37 | 99.45 | 99.01 | 98.94 | 99.03 |
| Linearity/% | 95.21 | 94.08 | 94.23 | 94.08 | 93.69 | 94.00 |

It can be seen from the results in Table II-2 and continued Table II-2 that, by using the solid acid catalyst of the present application, a long-term continuous operation of long-chain alkylbenzene preparation can be achieved through regeneration under an oxygen-containing mixed gas.

The preferred embodiments of the present application have been described in detail above. However, this application is not limited to the specific details in the above-mentioned embodiments. Within the scope of the technical concept of the present application, a variety of simple modifications can be made to the technical solutions of the present application. These simple modifications all fall within the protection scope of the present application.

In addition, it should be noted that each of the specific technical features described in the above-mentioned specific embodiments can be combined through any suitable manner without conflict. In order to avoid unnecessary repetition, various possible combinations will not be further described in the present application.

In addition, any combinations can be also made between various different embodiments of the present application, as long as they do not violate the spirit of the present application; and they should also be regarded as the inventions of the present application.

## Claims

1. A metal-modified molecular sieve, comprising a silica-alumina molecular sieve and a modifying metal selected from an alkaline earth metal, a Group IIIB metal, a Group IIIA metal, or combinations thereof, wherein at least a portion of the modifying metal exists in the form of an extra-framework compensation cation of the molecular sieve, and the content of the modifying metal as element ranges from 8 wt.% to 30 wt.%, and preferably from 12 wt.% to 23 wt.% based on the total amount of the metal-modified molecular sieve;
wherein, in the metal-modified molecular sieve, the ratio of the weight percentage of the surface-layer modifying metal measured by X-ray photoelectron spectroscopy (XPS) to the weight percentage of the bulk modifying metal measured by X-ray fluorescence (XRF) does not exceed 1.45, preferably ranges from 0.9 to 1.45, and more preferably from 0.9 to 1.35, calculated on the basis of the weight percentage of the element.

2. The metal-modified molecular sieve according to claim 1, wherein the modifying metal has an atomic radius ranging from 120 pm to 270 pm, and preferably from 160 pm 240 pm;
preferably, the modifying metal is selected from La, Ce, Sr, Y, or combinations thereof.

3. The metal-modified molecular sieve according to claim 1 or 2, wherein the metal-modified molecular sieve has a silica/alumina molar ratio ranging from 1:1 to 100:1, and preferably from 3:1 to 20:1;
preferably, the metal-modified molecular sieve is selected from X-type molecular sieve, Y-type molecular sieve, MCM-22-type molecular sieve, Beta-type molecular sieve, MOR-type molecular sieve, or combinations thereof, more preferably X-type molecular sieve and/or Y-type molecular sieve.

4. The metal-modified molecular sieve according to any one of claims 1-3, wherein the metal-modified molecular sieve has two absorption peaks in the range from 1440 cm⁻¹ to 1460 cm⁻¹ of the pyridine adsorption infrared spectrum, and preferably, the ratio of the area of the absorption peak at the lower wavenumber position to the total area of the two absorption peaks ranges from 0.3 to 0.7, and more preferably from 0.4 to 0.6.

5. A method for preparing the metal-modified molecular sieve according to any one of the preceding claims, comprising the following steps:
1) performing a first ion exchange of a molecular sieve precursor with a first exchange liquid containing a soluble compound of modifying metal;
2) subjecting the product of the first ion exchange to a first calcination to obtain a first exchanged molecular sieve;
3) performing a second ion exchange of the first exchanged molecular sieve with a second exchange liquid containing a soluble compound of modifying metal;
4) subjecting the product of the second ion exchange to a second calcination, and optionally repeating the second ion exchange and the second calcination one or more times to obtain a second exchanged molecular sieve;
5) heat-treating the second exchanged molecular sieve in an oxygen-containing atmosphere to obtain the metal-modified molecular sieve;
wherein, the first calcination and/or the second calcination is/are performed under an alkaline atmosphere,
preferably, the heat treatment in step 5) is performed at a higher temperature than the temperature of the second calcination in step 4), preferably at a temperature 50-100 °C higher than the temperature of the second calcination.

6. The preparation method according to claim 5, wherein the molecular sieve precursor is a hydrogen-type or sodium-type molecular sieve;
preferably, the molecular sieve precursor is a sodium-type molecular sieve, and the first exchange liquid and/or the second exchange liquid further contains an ammonium salt, more preferably, the ammonium salt is selected from ammonium nitrate, ammonium chloride, ammonium sulfate, or combinations thereof, further preferably, the concentrations of the ammonium salt in the first exchange liquid and/or the second exchange liquid each independently range from 50 g/L to 200 g/L.

7. The preparation method according to claim 5 or 6, having one or more of the following characteristics:
the soluble compounds of modifying metal contained in the first exchange solution and the second exchange solution may be identical or different, and are each independently selected from chloride, nitrate, phosphate, sulfate, or combinations thereof of the modifying metal;
the concentrations of the soluble compound of modifying metal in the first exchange liquid and/or the second exchange liquid each independently range from 100 g/L to 500 g/L;
the weight ratio of the first exchange liquid to the molecular sieve precursor ranges from 2:1 to 8:1, and the weight ratio of the second exchange liquid to the first exchanged molecular sieve ranges from 3:1 to 6:1;
the conditions of the first ion exchange include: the exchange temperature ranges from 50 °C to 90 °C, and preferably from 70 °C to 90 °C; the exchange time ranges from 0.5 h to 2 h, and preferably from 0.5 h to 1.5 h, and the exchange temperature of the second ion exchange is 0-15°C, and preferably 1-10°C higher than the exchange temperature of the first ion exchange, the exchange time ranges from 0.5 h to 2 h, and preferably 0.5 h to 1.5 h.

8. The preparation method according to any one of claims 5-7, having one or more of the following characteristics:
the alkaline atmosphere is provided by a vapor of an aqueous solution of an alkaline compound, preferably, the alkaline compound is selected from ammonia, ammonium carbonate, urea, or combinations thereof, more preferably, the alkaline compound is present in a concentration ranging 0.01 mol/L to 2 mol/L in the aqueous solution of the alkaline compound;
relative to 50 g of the molecular sieve precursor, the introduction rates of the alkaline atmosphere during the first and second calcinations, calculated on the volume of the aqueous solution of the alkaline compound, each independently ranges from 0.01 mL/min to 0.5 mL/min;
the conditions of the first calcination include: the calcination temperature ranges from 400 °C to 600 °C, the calcination time ranges from 0.5 h to 3 h, the pressure ranges from 0.01 MPa to 0.1 MPa (gauge pressure); and
the conditions of the second calcination include: the calcination temperature ranges from 400 °C to 600 °C, the calcination time ranges from 0.5 h to 3 h, the pressure ranges from 0.01 MPa to 0.1 MPa (gauge pressure).

9. The preparation method according to any one of claims 5-8, wherein:
the oxygen-containing atmosphere in step 5) is an air, or a mixture of an oxygen gas and an inert gas, preferably, the oxygen gas is present in an amount ranging from 16 vol.% to 30 vol.%, and preferably from 18 vol.% to 25 vol.% in the oxygen-containing atmosphere; and/or
the conditions of the heat treatment in step 5) include: the temperature ranges from 500 °C to 700 °C, and preferably from 550 °C to 650°C, and the treatment time ranges from 2 h to 12 h, and preferably from 4 h to 8 h.

10. A solid acid catalyst, comprising the metal-modified molecular sieve according to any one of claims 1-4 and a heat-resistant inorganic oxide, preferably, the weight ratio of the metal-modified molecular sieve to the heat-resistant inorganic oxide ranges from 99:1 to 20:80, preferably from 95:5 to 25:75, and more preferably from 90:10 to 50:50 on a dry basis.

11. The solid acid catalyst according to claim 10, wherein the heat-resistant inorganic oxide is selected from alumina, zirconia, silica, titania, clay, kaolin, montmorillonite, magnesia-alumina spinel, amorphous silica-alumina, or combinations thereof, preferably selected from alumina, zirconia, silica, titania, or combinations thereof;
preferably, the heat-resistant inorganic oxide has a particle size ranging from 0.005 µm to 200 µm, and further preferably from 0.01 µm to 100 µm.

12. A method for preparing the solid acid catalyst according to claim 10, comprising: mixing the metal-modified molecular sieve according to any one of claims 1-4, a heat-resistant inorganic oxide and/or a precursor thereof, water, an optional extrusion aid, and an optional peptizing agent, molding and calcining to produce the solid acid catalyst.

13. The preparation method according to claim 12, having one or more of the following characteristics:
the extrusion aid is selected from sesbania powder, cellulose, starch, or combinations thereof, preferably, the extrusion aid is used in an amount ranging from 0 wt.% to 20 wt.%, and more preferably from 1 wt.% to 5 wt.% based on the total weight of the metal-modified molecular sieve and the heat-resistant inorganic oxide and/or a precursor thereof on a dry basis;
the peptizing agent is selected from citric acid, nitric acid, phosphoric acid, or combinations thereof, preferably the peptizing agent is used in an amount ranging from 0 wt.% to 20 wt.%, and more preferably from 1 wt.% to 8 wt.% based on the total weight of the metal-modified molecular sieve and the heat-resistant inorganic oxide and/or a precursor thereof on a dry basis;
the ratio of the weight of the water used to the total weight of the metal-modified molecular sieve and the heat-resistant inorganic oxide and/or a precursor thereof on a dry basis ranges from 0.2:1 to 1.2:1, and preferably 0.3:1 to 1:1; and
the calcining temperature ranges from 400 °C to 650 °C, the calcining time ranges from 1 h to 12 h.

14. Use of the metal-modified molecular sieve according to any one of claims 1-4 or the solid acid catalyst according to claim 10 or 11 in the preparation of a long-chain hydrocarbon-based aromatic compound.

15. A method for preparing a long-chain hydrocarbon-based aromatic compound, comprising a step of reacting a long-chain olefin and an aromatic hydrocarbon in the presence of the metal-modified molecular sieve according to any one of claims 1-4,
preferably, the long-chain olefin is a long-chain alpha olefin having a double bond at the end, further preferably, the long-chain olefin is a C7-C27 long-chain olefin, still further preferably a C8-C15 long-chain olefin, and particularly preferably a C10-C14 long-chain olefin;
preferably, the aromatic hydrocarbon is a monocyclic or bicyclic aromatic hydrocarbon, more preferably a benzene-based aromatic hydrocarbon, still further preferably selected from benzene, toluene, ethylbenzene, or combinations thereof, particularly preferably benzene.

16. The method according to claim 15, wherein the reaction conditions include: the reaction temperature ranges from 50 °C to 250 °C, and preferably from 70 °C to 200 °C; the reaction pressure ranges from 0.1 MPa to 7 MPa, and preferably from 2 MPa to 4 MPa; the molar ratio of aromatic hydrocarbon/olefin ranges from 3:1 to 70:1, and preferably from 4:1 to 60:1; the weight space velocity of the long-chain olefin ranges from 0.1 h⁻¹ to 5 h⁻¹, and preferably from 0.3 h⁻¹ to 3 h⁻¹.

17. The method according to claim 15 or 16, wherein the reaction is carried out in the presence of the solid acid catalyst according to claim 10 or 11, preferably, the method further comprises a step of regenerating the solid acid catalyst in the presence of an oxygen-containing mixed gas;
further preferably, the oxygen-containing mixed gas is a mixture of an oxygen gas and a protective gas, and the protective gas is selected from nitrogen gas, helium gas, argon gas, or combinations thereof, more preferably, the oxygen gas is present in an amount ranging from 1 vol.% to 50 vol.%, and preferably from 10 vol.% to 25 vol.% in the oxygen-containing mixed gas;
still further preferably, the regeneration conditions include: the regeneration temperature ranges from 400 °C to 700 °C, the regeneration time ranges from 4 h to 18 h, the volume space velocity of the oxygen-containing mixed gas ranges from 500 h⁻¹ to 6000 h⁻¹, and the regeneration pressure ranges from 0.01 MPa to 0.1 MPa (gauge pressure).
